# EUROPEAN PATENT SPECIFICATION

(11) **EP 3 286 194 B1**
(45) Date of publication and mention of the grant of the patent: **04.12.2019**
(21) Application number: 16718324.3
(22) Date of filing: 21.04.2016
(51) Int. Cl.: C07D 487/04, A61K 31/53, A61P 25/00

(54) **IMIDAZOTRIAZINONES AS PDE1 INHIBITORS**
IMIDAZOTRIAZINONE ALS PDE1-INHIBITOREN
IMIDAZOTRIAZINONES UTILISÉES COMME INHIBITEURS DE PDE1

(30) Priority: 22.04.2015 DK 201500250; 04.04.2016 DK 201600201
(43) Date of publication of application: 28.02.2018
(73) Proprietor: H. Lundbeck A/S, 2500 Valby (DK)
(72) Inventor: KEHLER, Jan, 2800 Lyngby (DK); RASMUSSEN, Lars, Kyhn, 2720 Vanløse (DK); LANGGÅRD, Morten, 2600 Glostrup (DK); JESSING, Mikkel, 2000 Frederiksberg (DK); VITAL, Paulo, Jorge, Vieira, 1671 København V (DK); JUHL, Karsten, 2670 Greve (DK)
(74) Representative: H. Lundbeck A/S
(86) International application number: PCT/EP2016/058910
(87) International publication number: WO 2016/170064

(56) References cited:
- WO-A1-2012/040230
- WO-A1-2014/151409
- WO-A1-2016/055618
- WO-A1-2016/147659

## Description

### FIELD OF THE INVENTION

The present invention provides compounds that are PDE1 enzyme inhibitors and their use as a medicament, in particular for the treatment of neurodegenerative disorders and psychiatric disorders. The present invention also provides pharmaceutical compositions comprising compounds of the invention and methods of treating disorders using the compounds of the invention.

### BACKGROUND OF THE INVENTION

Throughout this application, various publications are referenced in full. The disclosures of these publications are hereby incorporated by reference into this application to describe more fully the state of the art to which this invention pertains.

The second messenger cyclic Nucleotides (cNs), cyclic adenosine monophosphate (cAMP) and cyclic guanosine monophosphate (cGMP) play a major role in intracellular signal transduction cascade, by regulating cN-dependent protein kinases (PKA and PKG), EPACs (Exchange Protein Activated by cAMP), phosphoprotein phosphatases, and/or cN-gated cation channels. In neurons, this includes the activation of cAMP- and cGMP-dependent kinases and subsequent phosphorylation of proteins involved in acute regulation of synaptic transmission as well as in neuronal differentiation and survival. Intracellular concentrations of cAMP and cGMP are strictly regulated by the rate of biosynthesis by cyclases and by the rate of degradation by phosphodiesterases (PDEs, EC 3.1.4.17). PDEs are bimetallic hydrolases that inactivate cAMP/cGMP by catalytic hydrolysis of the 3'-ester bond, forming the inactive 5'-monophosphate. Since PDEs provide the only means of degrading the cyclic nucleotides cAMP and cGMP in cells, PDEs play an essential role in cyclic nucleotide signalling. The catalytic activities of PDEs provide for breakdown of cNs over a spectrum of cN-concentrations in all cells, and their varied regulatory mechanisms provide for integration and crosstalk with myriad signalling pathways. Particular PDEs are targeted to discrete compartments within cells where they control cN level and sculpt microenvironments for a variety of cN signalosomes (Sharron H. Francis, Mitsi A. Blount, and Jackie D. Corbin. Physiol Rev 2011, 91: 651-690).

On the basis of substrate specificity, the PDE families can be divided into three groups: 1) The cAMP-specific PDEs, which include PDE4, PDE7, and PDE8, 2) the cGMP-selective enzymes PDE5 and PDE9, and 3) the dual-substrate PDEs, PDE1, PDE2, PDE3, as well as PDE10 and PDE11.

Previously named calmodulin-stimulated PDE (CaM-PDE), PDE1 is unique in that it is Ca²⁺-dependently regulated via calmodulin (CaM, a 16 kDa Ca²⁺-binding protein) complexed with four Ca²⁺ (for review, Sharron H. Francis, Mitsi A. Blount, and Jackie D. Corbin. Physiol Rev 2011, 91: 651-690). Thus, PDE1 represents an interesting regulatory link between cyclic nucleotides and intracellular Ca2+. The PDE1 family is encoded by three genes: PDE1A (mapped on human chromosome 2q32), PDE1B (human chromosome location, hcl: 12q13) and PDE1C (hcl: 7p14.3). They have alternative promoters and give rise to a multitude of proteins by alternative splicing which differ in their regulatory properties, substrate affinities, specific activities, activation constants for CaM, tissue distribution and molecular weights. More than 10 human isoforms are identified. Their molecular weights vary from 58 to 86 kDa per monomer. The N-terminal regulatory domain that contains two Ca²⁺/CaM binding domains and two phosphorylation sites differentiate their corresponding proteins and modulate their biochemical functions. PDE1 is a dual substrate PDE and the PDE1C-subtype has equal activity towards cAMP and cGMP (Km ≈ 1-3 µM), whereas the subtypes PDE1A and PDE1B have a preference for cGMP (Km for cGMP ≈ 1-3 µM and for cAMP ≈ 10-30 µM).

The PDE1 subtypes are highly enriched in the brain and located especially in the striatum (PDE1B), hippocampus (PDE1A) and cortex (PDE1A) and this localization is conserved across species (Amy Bernard et al. Neuron 2012, 73, 1083-1099). In the cortex, PDE1A is present mainly in deep cortical layers 5 and 6 (output layers), and used as a specificity marker for the deep cortical layers. PDE1 inhibitors enhance the levels of the second messenger cNs leading to enhanced neuronal excitability.

Thus, PDE1 is a therapeutic target for regulation of intracellular signalling pathways, preferably in the nervous system and PDE1 inhibitors can enhance the levels of the second messengers cAMP/cGMP leading to modulation of neuronal processes and to the expression of neuronal plasticity-related genes, neurotrophic factors, and neuroprotective molecules. These neuronal plasticity enhancement properties together with the modulation of synaptic transmission make PDE1 inhibitors good candidates as therapeutic agents in many neurological and psychiatric conditions. The evaluation of PDE1 inhibitors in animal models (for reviews see e.g. Blokland et al. Expert Opinion on Therapeutic Patents (2012), 22(4), 349-354; and Medina, A. E. Frontiers in Neuropharmacology (2011), 5(Feb.), 21) have suggested the potential for the therapeutic use of PDE1 inhibitors in neurological disorders, like e.g. Alzheimer's, Parkinson's and Huntington's Diseases and in psychiatric disorders like e.g. Attention Deficit hyperactivity Disorder (ADHD), restless leg syndrome, depression, anxiety, narcolepsy, cognitive impairment and cognitive impairment associated with schizophrenia (CIAS). There have also been patent applications claiming that PDE1 inhibitors are useful in diseases that may be alleviated by the enhancement of progesterone-signalling such as female sexual dysfunction (e.g. WO 2008/070095).

WO2012/040230 (Envivo Pharmaceuticals, Inc.) discloses imidazotriazinones as PDE9 inhibitors. WO2014/151409 discloses compounds as PDE1 inhibitors.

The compounds of the invention may offer alternatives to current marketed treatments for neurodegenerative and/or psychiatric disorders, treatments which are not efficacious in all patients. Hence, there remains a need for alternative methods of treatment of such diseases.

### SUMMARY OF THE INVENTION

PDE1 enzymes are expressed in the Central Nervous System (CNS), making this gene family an attractive source of new targets for the treatment of psychiatric and neurodegenerative disorders.

The objective of the present invention is to provide compounds that are PDE1 inhibitors, and as such are useful to treat neurodegenerative disorders and psychiatric disorders. Preferrably, said compounds are at least a ten-fold stronger as PDE1 inhibitors than as PDE9 inhibitors such that e.g. potentially unwanted effects associated with PDE9 inhibition can be prevented.

Accordingly, the present invention relates to compounds of formula (I) 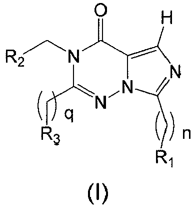 wherein
n is 0 or 1;
q is 0 or 1;
R1 is selected from the group consisting of linear or branched C₁-C₈ alkyl, saturated monocyclic C₃-C₈ cycloalkyl, tetrahydrofuranyl and tetrahydropyranyl; all of which can be substituted one or more times with fluorine;
R2 is selected from the group consisting of hydrogen, linear or branched C₁-C₈ alkyl, saturated monocyclic C₃-C₈ cycloalkyl, tetrahydrofuranyl and tetrahydropyranyl; or R2 is phenyl or 5 or 6-membered heteroaryl, all of which can be substituted with one or more substituents selected from the group consisting of halogen, C₁-C₃ alkyl and methoxy; or R2 is a saturated monocyclic C₃-C₈ cycloalkyl, substituted one or two times with methyl;
R3 is selected from the group consisting of hydrogen, C₁-C₅ alkyl, and phenyl; or
R3 is selected from the group consisting of C₁-C₅ alkyl substituted one or more times with fluorine; or
R3 is phenyl substituted one or more times with C₁-C₃ alkyl;
with the proviso that R2 and R3 cannot be hydrogen at the same time;
and tautomers and pharmaceutically acceptable addition salts thereof.

Reference to Compound I includes the free base of Compound I, pharmaceutically acceptable salts of Compound I, such as acid addition salts of Compound I, racemic mixtures of Compound I, or the corresponding enantiomer and/or optical isomer of Compound I, and polymorphic and amorphic forms of Compound I as well as tautomeric forms of Compound I. Furthermore, the compounds of this invention may exist in unsolvated as well as in solvated forms with pharmaceutically acceptable solvents such as water, ethanol and the like. In general, the solvated forms are considered equivalent to the unsolvated forms for the purposes of this invention.

In one embodiment, the invention relates to a compound according to formula (I) for use in therapy.

In one embodiment, the invention relates to a compound according to formula (I), for use in the treatment of a neurodegenerative disorder, selected from the group consisting of Alzheimer's Disease, Parkinson's Disease and Huntington's Disease or for the treatment of a psychiatric disorder such as Attention Deficit hyperactivity Disorder (ADHD), depression, anxiety, narcolepsy, cognitive impairment and cognitive impairment associated with schizophrenia (CIAS), or another brain disease like restless leg syndrome.

In one embodiment, the invention relates to a pharmaceutical composition comprising a compound according formula (I), and one or more pharmaceutically acceptable carrier or excipient.

In one embodiment, the invention relates to a compound of formula (I) for use in a method for the treatment of a neurodegenerative disorder, selected from the group consisting of Alzheimer's Disease, Parkinson's Disease and Huntington's Disease or for the treatment of a psychiatric disorder such as Attention Deficit hyperactivity Disorder (ADHD), depression, anxiety, narcolepsy, cognitive impairment and cognitive impairment associated with schizophrenia (CIAS), or another brain disease like restless leg syndrome, which method comprises the administration of a therapeutically effective amount of a compound according to formula (I) to a patient in need thereof.

### DETAILED DESCRIPTION OF THE INVENTION

### EMBODIMENTS OF THE INVENTION

The following notation is applied: an embodiment of the invention is identified as Ei, where i is an integer indicating the number of the embodiment. An embodiment Ei' specifying a specific embodiment a previously listed embodiment Ei is identified as Ei'(Ei), e.g. E2(E1) means "in an embodiment E2 of embodiment E1".

Where an embodiment is a combination of two embodiments the notation is similarly Ei"(Ei'and Ei), e.g. E3(E2 and E1) means "in an embodiment E3 of any of embodiments E2 and E1"

Where an embodiment is a combination of more than two embodiments the notation is similarly Ei"'(Ei), Ei' and Ei"), e.g. E4(E1, E2 and E3) means "in an embodiment E4 of any of embodiments E1, E2 and E3"

In a first embodiment E1 the present invention relates to compounds of formula (I) (Compound I) 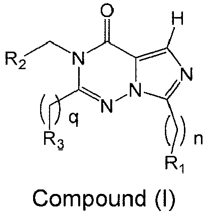 wherein
n is 0 or 1;
q is 0 or 1;
R1 is selected from the group consisting of linear or branched C₁-C₈ alkyl, saturated monocyclic C₃-C₈ cycloalkyl, tetrahydrofuranyl and tetrahydropyranyl; all of which can be substituted one or more times with fluorine;
R2 is selected from the group consisting of hydrogen, linear or branched C₁-C₈ alkyl, saturated monocyclic C₃-C₈ cycloalkyl, tetrahydrofuranyl and tetrahydropyranyl; or
R2 is phenyl or 5 or 6-membered heteroaryl, all of which can be substituted with one or more substituents selected from the group consisting of halogen, C₁-C₃ alkyl and methoxy; or
R2 is a saturated monocyclic C₃-C₈ cycloalkyl, substituted one or two times with methyl;
R3 is selected from the group consisting of hydrogen, C₁-C₅ alkyl, and phenyl; or
R3 is selected from the group consisting of C₁-C₅ alkyl substituted one or more times with fluorine; or
R3 is phenyl substituted one or more times with C₁-C₃ alkyl;
with the proviso that R2 and R3 cannot be hydrogen at the same time;
and tautomers and pharmaceutically acceptable addition salts thereof.

E2(E1) n is 0 and q is 0;
   R₁ is selected from tetrahydrofuranyl and tetrahydropyranyl;
   R₂ is selected from the group consisting of, linear or branched C₁-C₈ alkyl, phenyl, saturated monocyclic C₃-C₈ cycloalkyl, tetrahydrofuranyl and tetrahydropyranyl; or
   R₂ is selected from the group consisting of phenyl substituted with a substituent selected from the group consisting of halogen, C₁-C₃ alkyl and methoxy;
   R₃ is selected from the group consisting of hydrogen and C₁-C₅ alkyl; or
   R₃ is methyl or ethyl substituted one, two or three times with fluorine;
E3(E1 or E2) n is 0 and R₁ is tetrahydropyranyl.
E4(E1 or E2) R₂ is phenyl.
E5(E1 or E2) R₂ is substituted phenyl, wherein the substituent is selected from the group consisting of fluorine, chlorine, methyl and methoxy.
E6(E1 or E2) R₂ is saturated monocyclic C₃-C₈ cycloalkyl.
E7(E1 or E2) R₂ is saturated monocyclic C₃-C₈ cycloalkyl substituted with methyl.
E8(E1 or E2) R₂ is C₁-C₃ alkyl.
E9(E1 or E2) R₂ is methyl, ethyl or isopropyl
E10(E1 or E2) R₂ is tetrahydrofuranyl
E11(E1 or E2) R₂ is tetrahydropyranyl
E12(E1 or E2) n is 0.
E13(E1 or E2) n is 1.
E14(E1 or E2) q is 0.
E15(E1 or E2) q is 1.
E18 (E1 or E2) R₃ is hydrogen
E19(E1 or E2) R₃ is C₁-C₃ alkyl
E20(E19) R₃ is methyl or ethyl
E21(E1) R3 is hydrogen, methyl or ethyl;
   and R2 is selected from the group consisting of linear or branched C₁-C₈ alkyl, saturated monocyclic C₃-C₈ cycloalkyl, tetrahydrofuranyl and tetrahydropyranyl; or
   R2 is phenyl or 5 or 6-membered heteroaryl, all of which can be substituted with one or more substituents selected from the group consisting of halogen, C₁-C₃ alkyl and methoxy; or
   R2 is a saturated monocyclic C₃-C₈ cycloalkyl, substituted one or two times with methyl.
E22(E1) Q is 0 and R3 is benzyl substituted with methyl;
   and R2 is selected from the group consisting of linear or branched C₁-C₈ alkyl, saturated monocyclic C₃-C₈ cycloalkyl, tetrahydrofuranyl and tetrahydropyranyl; or
   R2 is phenyl or 5 or 6-membered heteroaryl, all of which can be substituted with one or more substituents selected from the group consisting of halogen, C₁-C₃ alkyl and methoxy; or
   R2 is a saturated monocyclic C₃-C₈ cycloalkyl, substituted one or two times with methyl;
E23(E1), the compound of formula (I) is selected among the compounds listed in Table 1, in the form of the free base, one or more tautomers thereof or a pharmaceutically acceptable salt thereof.
E24(E1 to E26) the compound has a PDE1A, PDE1B or PDE1C IC₅₀ value, determined as described in the section "PDE1 inhibition assay", of 10 micro molar or less, such as 5 micro molar or less, such as 4 micro molar or less, such as 3 micro molar or less, such as 2 micro molar or less, such as 1 micro molar or less, such as 500 nM or less, such as 400 nM or less, such as 300 nM or less, such as 200 nM or less, such as 100 nM or less.
E25(E1) the compound is selected from the compounds listed in Table 1 and pharmaceutically acceptable salts thereof.
E26(E1 to E25) the compound is for use as a medicament.
E27 (E1 to E25) a pharmaceutical composition comprising a therapeutically effective amount of a compound of any of embodiments (E1) to (E26), and one or more pharmaceutically acceptable carriers, diluents and excipients.
E28(E1 to E25) a compound of any of embodiments (E1) to (E25) for use in the treatment of neurodegenerative disorder, selected from the group consisting of Alzheimer's Disease, Parkinson's Disease and Huntington's Disease or for the treatment of a psychiatric disorder such as Attention Deficit hyperactivity Disorder (ADHD), depression, anxiety, narcolepsy, cognitive impairment and cognitive impairment associated with schizophrenia (CIAS) or another brain disease such as restless leg syndrome.
E29(E27) the pharmaceutical composition is for the treatment of neurodegenerative disorder, selected from the group consisting of Alzheimer's Disease, Parkinson's Disease and Huntington's Disease or for the treatment of a psychiatric disorder such as Attention Deficit hyperactivity Disorder (ADHD), depression, anxiety, narcolepsy, cognitive impairment and cognitive impairment associated with schizophrenia (CIAS), or another brain disease such as restless leg syndrome.
E30(E1 to E25) a compound of formula (I) for use in a method of treating a subject suffering from neurodegenerative disorder, selected from the group consisting of Alzheimer's Disease, Parkinson's Disease and Huntington's Disease or for the treatment of a psychiatric disorder such as Attention Deficit hyperactivity Disorder (ADHD), depression, anxiety, narcolepsy, cognitive impairment and cognitive impairment associated with schizophrenia (CIAS), or another brain disease like restless leg syndrome, which method comprises administering to said subject an amount of a compound of any of embodiments (E1) to (E25).
E31(E1 to E25) use of a compound of any of embodiments (E1) to (E25) in the manufacture of a medicament for the treatment of a neurodegenerative disorder, selected from the group consisting of Alzheimer's Disease, Parkinson's Disease and Huntington's Disease or for the treatment of a psychiatric disorder such as Attention Deficit hyperactivity Disorder (ADHD), depression, anxiety, narcolepsy, cognitive impairment and cognitive impairment associated with schizophrenia (CIAS) or another brain disease like restless leg syndrome.

### DEFINITIONS

### PDE1 ENZYMES

The PDE1 isozyme family includes numerous splice variant PDE1 isoforms. It has three subtypes, PDE1A, PDE1B and PDE1C which divide further into various isoforms. In the context of the present invention PDE1 and PDE1 enzymes are synonymous and refer to PDE1A, PDE1B and PDE1C enzymes as well as their isoforms unless otherwise specified.

### SUBSTITUENTS

As used in the context of the present invention, the terms "halo" and "halogen" are used interchangeably and refer to fluorine, chlorine, bromine or iodine.

A given range may interchangeably be indicated with "-" (dash) or to , e.g. the term "C₁-C₃ alkyl" is equivalent to "C₁ to C₃ alkyl".

The terms "C₁-C₃ alkyl", "C₁-C₄ alkyl", "C₁-C₅ alkyl", "C₁-C₆ alkyl", "C₁-C₇ alkyl" and "C₁-C₈ alkyl" refer to a linear (i.e. unbranched) or branched saturated hydrocarbon having from one up to eight carbon atoms, inclusive. Examples of such groups include, but are not limited to, methyl, ethyl, 1-propyl, 2-propyl, 1-butyl, 2-butyl, 2-methyl-2-propyl, 2-methyl-1-butyl, n-hexyl, n-heptyl and n-octyl.

The term "saturated monocyclic C₃ to C₈ cycloalkyl" refers to cyclopropyl, cyclobutyl, cyclopentyl, cyclohexyl, cycloheptyl and cyclooctyl.

The term "heteroaryl" is intended to indicate a 5 or 6 membered aromatic monocyclic ring containing 1 to 5 carbon atoms and one or more heteroatoms selected from oxygen, nitrogen and sulfur. In a preferred embodiment, a 6-membered heteryaryl is pyridinyl. In a preferred embodiment, a 5-membered heteroaryl is thiophenyl.

### ISOMERIC FORMS

Where compounds of the present invention contain one or more chiral centers reference to any of the compounds will, unless otherwise specified, cover the enantiomerically or diastereomerically pure compound as well as mixtures of the enantiomers or diastereomers in any ratio.

The above also applies where compounds of the invention contain more than two chiral centers.

### PDE1 INHIBITORS AND PDE9 INHIBITORS

In the context of the present invention a compound is considered to be a PDE1 inhibitor if the amount required to reach the IC₅₀ level of any of the three PDE1 isoforms is 10 micro molar or less, preferably less than 9 micro molar, such as 8 micro molar or less, such as 7 micro molar or less, such as 6 micro molar or less, such as 5 micro molar or less, such as 4 micro molar or less, such as 3 micro molar or less, more preferably 2 micro molar or less, such as 1 micro molar or less, in particular 500 nM or less. In preferred embodiments the required amount of PDE1 inhibitor required to reach the IC₅₀ level of PDE1B is 400nM or less, such as 300 nM or less, 200nM or less, 100 nM or less, or even 80 nM or less, such as 50 nM or less, for example 25 nM or less.

In a preferred embodiment the compounds of the present invention are at least a ten-fold stronger PDE1 inhibitors than PDE9 inhibitors, i.e. the amount of the compound required to reach the IC₅₀ level of one or more of the three PDE1 isoforms is at least a ten-fold less than the amount of the same compound required to reach the IC₅₀ level of the PDE9 enzyme.

### PHARMACEUTICALLY ACCEPTABLE SALTS

The present invention also comprises salts of the compounds, typically, pharmaceutically acceptable salts. Such salts include pharmaceutically acceptable acid addition salts. Acid addition salts include salts of inorganic acids as well as organic acids.

Representative examples of suitable inorganic acids include hydrochloric, hydrobromic, hydroiodic, phosphoric, sulfuric, sulfamic, nitric acids and the like. Representative examples of suitable organic acids include formic, acetic, trichloroacetic, trifluoroacetic, propionic, benzoic, cinnamic, citric, fumaric, glycolic, itaconic, lactic, methanesulfonic, maleic, malic, malonic, mandelic, oxalic, picric, pyruvic, salicylic, succinic, methane sulfonic, ethanesulfonic, tartaric, ascorbic, pamoic, bismethylene salicylic, ethanedisulfonic, gluconic, citraconic, aspartic, stearic, palmitic, EDTA, glycolic, p-aminobenzoic, glutamic, benzenesulfonic, p-toluenesulfonic acids, theophylline acetic acids, as well as the 8-halotheophyllines, for example 8-bromotheophylline and the like. Further examples of pharmaceutically acceptable inorganic or organic acid addition salts include the pharmaceutically acceptable salts listed in Berge, S.M. et al., J. Pharm. Sci. 1977, 66, 2, the contents of which are hereby incorporated by reference.

Furthermore, the compounds of this invention may exist in unsolvated as well as in solvated forms with pharmaceutically acceptable solvents such as water, ethanol and the like. In general, the solvated forms are considered equivalent to the unsolvated forms for the purposes of this invention.

### THERAPEUTICALLY EFFECTIVE AMOUNT

In the present context, the term "therapeutically effective amount" of a compound means an amount sufficient to cure, alleviate or partially arrest the clinical manifestations of a given disease and its complications in a therapeutic intervention comprising the administration of said compound. An amount adequate to accomplish this is defined as "therapeutically effective amount". Effective amounts for each purpose will depend on the severity of the disease or injury as well as the weight and general state of the subject. It will be understood that determining an appropriate dosage may be achieved using routine experimentation, by constructing a matrix of values and testing different points in the matrix, which is all within the ordinary skills of a trained physician.

In the present context, the term "treatment" and "treating" means the management and care of a patient for the purpose of combating a condition, such as a disease or a disorder. The term is intended to include the full spectrum of treatments for a given condition from which the patient is suffering, such as administration of the active compound to alleviate the symptoms or complications, to delay the progression of the disease, disorder or condition, to alleviate or relief the symptoms and complications, and/or to cure or eliminate the disease, disorder or condition as well as to prevent the condition, wherein prevention is to be understood as the management and care of a patient for the purpose of combating the disease, condition, or disorder and includes the administration of the active compounds to prevent the onset of the symptoms or complications. Nonetheless, prophylactic (preventive) and therapeutic (curative) treatments are two separate aspects of the invention. The patient to be treated is preferably a mammal, in particular a human being.

### PHARMACEUTICAL COMPOSITIONS

The present invention further provides a pharmaceutical composition comprising a therapeutically effective amount of a compound of formula (I) and a pharmaceutically acceptable carrier or diluent. The present invention also provides a pharmaceutical composition comprising a therapeutically effective amount of one of the specific compounds disclosed in the Experimental Section herein and a pharmaceutically acceptable carrier or diluent.

The compounds of the invention may be administered alone or in combination with pharmaceutically acceptable carriers, diluents or excipients, in either single or multiple doses. The pharmaceutical compositions according to the invention may be formulated with pharmaceutically acceptable carriers or diluents as well as any other known adjuvants and excipients in accordance with conventional techniques such as those disclosed in Remington: The Science and Practice of Pharmacy, 21st Edition, Gennaro, Ed., Mack Publishing Co., Easton, PA, 2005.

The pharmaceutical compositions may be specifically formulated for administration by any suitable route such as oral, rectal, nasal, pulmonary, topical (including buccal and sublingual), transdermal and parenteral (including subcutaneous, intramuscular and intravenous) routes. It will be appreciated that the route will depend on the general condition and age of the subject to be treated, the nature of the condition to be treated and the active ingredient.

Pharmaceutical compositions for oral administration include solid dosage forms such as capsules, tablets, dragees, pills, lozenges, powders and granules. Where appropriate, the compositions may be prepared with coatings such as enteric coatings or they may be formulated so as to provide controlled release of the active ingredient such as sustained or prolonged release according to methods well known in the art. Liquid dosage forms for oral administration include solutions, emulsions, suspensions, syrups and elixirs.

Pharmaceutical compositions for parenteral administration include sterile aqueous and nonaqueous injectable solutions, dispersions, suspensions or emulsions as well as sterile powders to be reconstituted in sterile injectable solutions or dispersions prior to use. Other suitable administration forms include, but are not limited to, suppositories, sprays, ointments, creams, gels, inhalants, dermal patches and implants.

Typical oral dosages range from about 0.001 to about 100 mg/kg body weight per day. Typical oral dosages also range from about 0.01 to about 50 mg/kg body weight per day. Typical oral dosages further range from about 0.05 to about 10 mg/kg body weight per day. Oral dosages are usually administered in one or more dosages, typically, one to three dosages per day. The exact dosage will depend upon the frequency and mode of administration, the sex, age, weight and general condition of the subject treated, the nature and severity of the condition treated and any concomitant diseases to be treated and other factors evident to those skilled in the art.

The formulations may also be presented in a unit dosage form by methods known to those skilled in the art. For illustrative purposes, a typical unit dosage form for oral administration may contain from about 0.01 to about 1000 mg, from about 0.05 to about 500 mg, or from about 0.5 mg to about 200 mg.

The present invention also provides a process for making a pharmaceutical composition comprising mixing a therapeutically effective amount of a compound of formula (I) and at least one pharmaceutically acceptable carrier or diluent. In an embodiment, of the present invention, the compound utilized in the aforementioned process is one of the specific compounds disclosed in the Experimental Section herein.

The compounds of this invention are generally utilized as the free substance or as a pharmaceutically acceptable salt thereof. One example is an acid addition salt of a compound which has the same utility as of a free base. When a compound of formula (I) contains a free base such salts are prepared in a conventional manner by treating a solution or suspension of a free base of formula (I) with a pharmaceutically acceptable acid. Representative examples of suitable organic and inorganic acids are described above.

For parenteral administration, solutions of the compounds of formula (I) in sterile aqueous solution, aqueous propylene glycol or sesame or peanut oil may be employed. Such aqueous solutions should be suitably buffered if necessary and the liquid diluent first rendered isotonic with sufficient saline or glucose. The aqueous solutions are particularly suitable for intravenous, intramuscular and subcutaneous administration. The compounds of formula (I) may be readily incorporated into known sterile aqueous media using standard techniques known to those skilled in the art.

Suitable pharmaceutical carriers include inert solid diluents or fillers, sterile aqueous solutions and various organic solvents. Examples of solid carriers include lactose, terra alba, sucrose, cyclodextrin, talc, gelatin, agar, pectin, acacia, magnesium stearate, stearic acid and lower alkyl ethers of cellulose. Examples of liquid carriers include, but are not limited to, syrup, peanut oil, olive oil, phospholipids, fatty acids, fatty acid amines, polyoxyethylene and water. Similarly, the carrier or diluent may include any sustained release material known in the art, such as glyceryl monostearate or glyceryl distearate, alone or mixed with a wax. The pharmaceutical compositions formed by combining the compounds of formula (I) and a pharmaceutically acceptable carrier are then readily administered in a variety of dosage forms suitable for the disclosed routes of administration. The formulations may conveniently be presented in unit dosage form by methods known in the art of pharmacy.

Formulations of the present invention suitable for oral administration may be presented as discrete units such as capsules or tablets, each containing a predetermined amount of the active ingredient, and optionally a suitable excipient. Furthermore, the orally available formulations may be in the form of a powder or granules, a solution or suspension in an aqueous or non-aqueous liquid, or an oil-in-water or water-in-oil liquid emulsion.

If a solid carrier is used for oral administration, the preparation may be tableted, placed in a hard gelatin capsule in powder or pellet form or it may be in the form of a troche or lozenge. The amount of solid carrier will vary widely but will range from about 25 mg to about 1 g per dosage unit. If a liquid carrier is used, the preparation may be in the form of a syrup, emulsion, soft gelatin capsule or sterile injectable liquid such as an aqueous or non-aqueous liquid suspension or solution.

The pharmaceutical compositions of the invention may be prepared by conventional methods in the art. For example, tablets may be prepared by mixing the active ingredient with ordinary adjuvants and/or diluents and subsequently compressing the mixture in a conventional tableting machine prepare tablets. Examples of adjuvants or diluents comprise: corn starch, potato starch, talcum, magnesium stearate, gelatin, lactose, gums, and the like. Any other adjuvants or additives usually used for such purposes such as colorings, flavorings, preservatives etc. may be used provided that they are compatible with the active ingredients.

### TREATMENT OF DISORDERS

As mentioned above, the compounds of formula (I) are PDE1 enzyme inhibitors and as such are useful to treat associated neurological and psychiatric disorders.

The invention thus provides a compound of formula (I) or a pharmaceutically acceptable acid addition salt thereof, as well as a pharmaceutical composition containing such a compound, for use in the treatment of a brain disease which could be a neurodegenerative disorder or a psychiatric disorder. In a preferred embodiment the neurodegenerative disorder is selected from the group consisting of Alzheimer's Disease, Parkinson's Disease and Huntington's Disease. In another preferred embodiment the psychiatric disorder is selected from the group consisting of Attention Deficit hyperactivity Disorder (ADHD), depression, anxiety, narcolepsy, cognitive impairment and cognitive impairment associated with schizophrenia (CIAS). Other brain disorders could be e.g. restless leg syndrome.

The present invention provides a compound of formula (I) for use in a method of treating a mammal, including a human, suffering from a neurodegenerative disorder selected from the group consisting of Alzheimer's Disease, Parkinson's Disease and Huntington's Disease, which method comprises administering to the subject a therapeutically effective amount of a compound of formula (I).

This invention further provides a compound of formula (I) for use in a method of treating a neurodegenerative disorder in a mammal, including a human, which method comprises administering to said mammal an amount of a compound of formula (I) effective in inhibiting PDE1.

This invention also provides a compound of formula (I) for use in a method of treating a subject suffering from a psychiatric disorder, which method comprises administering to the subject a therapeutically effective amount of a compound of formula (I). Examples of psychiatric disorders that can be treated according to the present invention include Attention Deficit hyperactivity Disorder (ADHD), depression, anxiety, narcolepsy, cognitive impairment and cognitive impairment associated with schizophrenia (CIAS).

This invention also provides a compound of formula (I) for use in a method of treating a subject suffering from a brain disorder such as restless leg syndrome.

Further, the invention is directed to the use of a compound of formula (I) in the manufacture of a medicament for the treatment of a neurodegenerative disorder, such as Alzheimer's Disease, Parkinson's Disease and Huntington's Disease or for the treatment of a psychiatric disorder such as Attention Deficit hyperactivity Disorder (ADHD), depression, narcolepsy, cognitive impairment and cognitive impairment associated with schizophrenia (CIAS). Further, the invention is directed to the use of a compound of formula (I) in the manufacture of a medicament for the treatment of a brain disease, such as restless leg syndrome The invention is also directed to a compound of formula (I) for use as a medicine. In a specific embodiment the compound of formula (I) for use in the treatment of a neurodegenerative disorder, such as Alzheimer's Disease, Parkinson's Disease and Huntington's Disease or for the treatment of a psychiatric disorder such as Attention Deficit hyperactivity Disorder (ADHD), depression, anxiety, narcolepsy cognitive impairment and cognitive impairment associated with schizophrenia (CIAS) or for the treatment of another brain disease like restless leg syndrome.

All references, including publications, patent applications and patents, cited herein are hereby incorporated by reference in their entirety and to the same extent as if each reference were individually and specifically indicated to be incorporated by reference and were set forth in its entirety (to the maximum extent permitted by law).

Headings and sub-headings are used herein for convenience only, and should not be construed as limiting the invention in any way.

The use of any and all examples, or exemplary language (including "for instance", "for example", "e.g.", and "as such") in the present specification is intended merely to better illuminate the invention, and does not pose a limitation on the scope of invention unless otherwise indicated.

The citation and incorporation of patent documents herein is done for convenience only, and does not reflect any view of the validity, patentability and/or enforceability of such patent documents.

### COMPOUNDS OF THE INVENTION

**Table 1: Compounds of the invention**

| **Example** | **Compound** | **PDE1A, IC₅₀ (nM)** | **PDE1B, IC₅₀ (nM)** | **PDE1C, IC₅₀ (nM)** | **% inhibition of PDE9 at 10 microM** |
|---|---|---|---|---|---|
| 1 | 3-(Cyclohexylmethyl)-2-methyl-7-(tetrahydro-2*H*-pyran-4-yl)imidazo[5,1-*f*][1,2,4]triazin-4(3*H*)-one | 15 | 30 | 9 | 26 |
| 2 | 3-Methyl-2-(4-methylbenzyl)-7-(tetrahydro-2*H*-pyran-4-yl)imidazo[5,1-*f*][1,2,4]triazin-4(3*H*)-one | 732 | 258 | 984 | 4 |
| 3 | 3-Ethyl-2-(4-methylbenzyl)-7-(tetrahydro-2*H*-pyran-4-yl)imidazo[5,1-*f*][1,2,4]triazin-4(3*H*)-one | 180 | 79 | 279 | 15 |
| 4 | 2-(4-Methylbenzyl)-3-propyl-7-(tetrahydro-2*H*-pyran-4-yl)imidazo[5,1-*f*][1,2,4]triazin-4(3*H*)-one | 83 | 40 | 61 | 9 |
| 5 | 3-Isobutyl-2-(4-methylbenzyl)-7-(tetrahydro-2*H*-pyran-4-yl)imidazo[5,1-*f*][1,2,4]triazin-4(3*H*)-one | 47 | 52 | 38 | -3 |
| 6 | 3-(Cyclopentylmethyl)-2-(4-methylbenzyl)-7-(tetrahydro-2*H-*pyran-4-yl)imidazo[5,1-*f*][1,2,4]triazin-4(3*H*)-one | 29 | 63 | 4 | 2 |
| 7 | 3-(Cyclohexylmethyl)-2-(4-methylbenzyl)-7-(tetrahydro-2*H-*pyran-4-yl)imidazo[5,1-*f*][1,2,4]triazin-4(3*H*)-one | 40 | 26 | 11 | 11 |
| 8 | 3-(Cyclopropylmethyl)-2-(4-methylbenzyl)-7-(tetrahydro-2*H-*pyran-4-yl)imidazo[5,1-*f*][1,2,4]triazin-4(3*H*)-one | 63 | 103 | 78 | 9 |
| 9 | 2-(4-Methylbenzyl)-7-(tetrahydro-2*H*-pyran-4-yl)-3-((tetrahydro-2*H-*pyran-4-yl)methyl)imidazo[5,1-*f*][1,2,4]triazin-4(3*H*)-one | 583 | 364 | 23 | 12 |
| 10 | 3-(Cyclobutylmethyl)-2-(4-methylbenzyl)-7-(tetrahydro-2*H-*pyran-4-yl)imidazo[5,1-*f*][1,2,4]triazin-4(3*H*)-one | 245 | 103 | 62 | -11 |
| 11 Stereoisomer 1 | *cis*-2-(4-Methylbenzyl)-3-((4-methylcyclohexyl)methyl)-7-(tetrahydro-2*H*-pyran-4-yl)imidazo[5,1-*f*][1,2,4]triazin-4(3*H*)-one | 43 | 33 | 69 | -9 |
| 11 Stereoisomer 2 | *trans*-2-(4-Methylbenzyl)-3-((4-methyl cyclohexyl)methyl)-7-(tetrahydro-2*H*-pyran-4-yl)imidazo[5,1-*f*][1,2,4]triazin-4(3*H*)-one | 8 | 10 | 5 | 15 |
| 12 Stereoisomer 1 | (-)-2-(4-Methylbenzyl)-7-(tetrahydro-2*H*-pyran-4-yl)-3-((tetrahydrofuran-3-yl)methyl)imidazo[5,1-*f*][1,2,4]triazin-4(3*H*)-one | n.d. | 258 | 105 | 3 |
| 12 Stereoisomer 2 | (+)-2-(4-Methylbenzyl)-7-(tetrahydro-2H-pyran-4-yl)-3-((tetrahydrofuran-3-yl)methyl)imidazo[5,1-*f*][1,2,4]triazin-4(3*H*)-one | 100 | 67 | 151 | 7 |
| 13 | 3-(3-Fluorobenzyl)-2-methyl-7-(tetrahydro-2*H*-pyran-4-yl)imidazo[5,1-*f*][1,2,4]triazin-4(3*H*)-one | 127 | 258 | 29 | -4 |
| 14 | 3-(4-Fluorobenzyl)-2-methyl-7-(tetrahydro-2H-pyran-4-yl)imidazo[5,1-*f*][1,2,4]triazin-4(3*H*)-one | 77 | 134 | 35 | 18 |
| 15 | 3-Benzyl-2-methyl-7-(tetrahydro-2*H*-pyran-4-yl)imidazo[5,1-*f*][1,2,4]triazin-4(3*H*)-one | 70. | 162 | 17 | -13 |
| 16 | 3-(2-Chlorobenzyl)-2-methyl-7-(tetrahydro-2*H*-pyran-4-yl)imidazo[5,1-*f*][1,2,4]triazin-4(3*H*)-one | 118 | 220 | 20 | 11 |
| 17 | 3-(3-Chlorobenzyl)-2-methyl-7-(tetrahydro-2*H*-pyran-4-yl)imidazo[5,1-*f*][1,2,4]triazin-4(3*H*)-one | 63 | 159 | 14 | 5 |
| 18 | 2-Methyl-3-(3-methylbenzyl)-7-(tetrahydro-2*H*-pyran-4-yl)imidazo[5,1-*f*][1,2,4]triazin-4(3*H*)-one | 265 | 188 | 20 | -30 |
| 19 | 3-(3-Methoxybenzyl)-2-methyl-7-(tetrahydro-2*H*-pyran-4-yl)imidazo[5,1-*f*][1,2,4]triazin-4(3*H*)-one | 115 | 259 | 52 | 4 |
| 20 | 3-(2-Fluorobenzyl)-2-methyl-7-(tetrahydro-2*H*-pyran-4-yl)imidazo[5,1-*f*][1,2,4]triazin-4(3*H*)-one | 54 | 139 | 15 | 21 |
| 21 | 2-Methyl-3-(2-methylbenzyl)-7-(tetrahydro-2*H*-pyran-4-yl)imidazo[5,1-*f*][1,2,4]triazin-4(3*H*)-one | 367 | 454 | 37 | 13 |
| 22 | 2-Methyl-3-(4-methylbenzyl)-7-(tetrahydro-2*H*-pyran-4-yl)imidazo[5,1-*f*][1,2,4]triazin-4(3*H*)-one | 58 | 66 | 77 | 18 |
| 23 | 3-(4-Methoxybenzyl)-2-methyl-7-(tetrahydro-2*H*-pyran-4-yl)imidazo[5,1-*f*][1,2,4]triazin-4(3*H*)-one | 29 | 26 | 21 | -4 |
| 24 | 3-(4-Chlorobenzyl)-2-methyl-7-(tetrahydro-2*H*-pyran-4-yl)imidazo[5,1-*f*][1,2,4]triazin-4(3*H*)-one | 35 | 61 | 104 | -11 |
| 25 | 2-Ethyl-3-(3-fluorobenzyl)-7-(tetrahydro-2*H*-pyran-4-yl)imidazo[5,1-*f*][1,2,4]triazin-4(3*H*)-one | 52 | 131 | 15 | 8 |
| 26 | 3-(Cyclohexylmethyl)-7-(tetrahydro-2*H*-pyran-4-yl)imidazo[5,1-*f*][1,2,4]triazin-4(3*H*)-one | 554 | 376 | 103 | 8 |
| 27 | 3-(3-Fluorobenzyl)-7-(tetrahydro-2*H*-pyran-4-yl)imidazo[5,1-*f*][1,2,4]triazin-4(3*H*)-one | n.d. | n.d. | 436 | 17 |
| 28 | 3-(Cyclopentylmethyl)-7-(tetrahydro-2*H*-pyran-4-yl)imidazo[5,1-*f*][1,2,4]triazin-4(3*H*)-one | n.d. | n.d. | 86 | 10 |
| 29 | 3-(Cycloheptylmethyl)-7-(tetrahydro-2*H*-pyran-4-yl)imidazo[5,1-*f*][1,2,4]triazin-4(3*H*)-one | 33 | 52 | 25 | 21 |

| | | | | | |
|---|---|---|---|---|---|
| nd means "not determined" | | | | | |

Table 1 lists the IC₅₀ value for inhibition of PDE1 by the compounds of the invention. The IC₅₀ value refers to the concentration (nM) of the compound required to reach 50% inhibition of the PDE1 enzyme at the specified substrate concentration.

For comparative purpose, the table also lists % inhibiton of PDE9 at 10 µM.

PDE1 and PDE9 assays are described in the Experimental Section.

### EXPERIMENTAL SECTION

### PREPARATION OF THE COMPOUNDS OF THE INVENTION

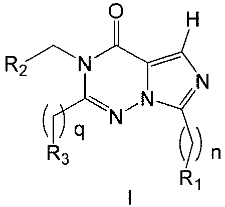

The compounds of formula (I) may be prepared by methods described below, together with synthetic methods known in the art of organic chemistry, or modifications that are familiar to those of ordinary skill in the art. The starting materials used herein are available commercially or may be prepared by routine methods known in the art, such as those method described in standard reference books such as "Compendium of Organic Synthetic Methods, Vol. I-XII" (published with Wiley-Interscience). Preferred methods include, but are not limited to, those described below.

The schemes are representative of methods useful in synthesizing the compounds of the present invention. They are not to constrain the scope of the invention in any way. Unless otherwise indicated, in the reaction schemes and discussion that follow, R1-R3 are as defined in claim 1.

### General Methods:

### Method 1:

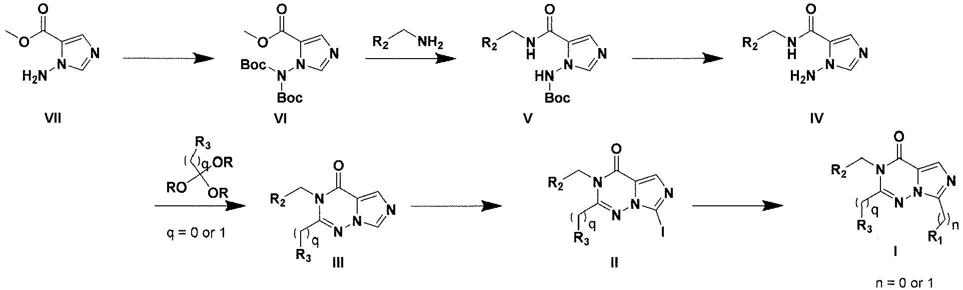

In brief, compounds **I** of the invention can be prepared from the commercially available methyl 1-amino-1*H*-imidazole-5-carboxylate (CAS 865444-80-0). Reacting methyl 1-amino-1*H*-imidazole-5-carboxylate with di-*tert*-butyl dicarbonate using a catalyst such as *N,N-*dimethylpyridin-4-amine, a base exemplified by but not limited to triethylamine in a solvent such as dichloromethane yields **VI.** Reacting intermediate **VI** with an amine using a suitable base exemplified by but not limited to potassium carbonate in a solvent such as methanol yields **V.** Intermediate **V** can be deprotected to yield the intermediate **IV** by treatment with an acid exemplified by but not limited to trifluoroacetic acid in a solvent such as THF. Intermediate **IV** can be further reacted with a carboxylic acid orthoester using a base exemplified but not limited to cesiumcarbonate to yield imidazo[5,1-*f*][1,2,4]triazin-4(3*H*)-ones **III.** Imidazo[5,1-*f*][1,2,4]triazin-4(3*H*)-ones **II** are prepared by treatment of **III** by a strong base, exemplified by but not limited to 2,2,6,6-tetramethylpiperidinylmagnesium chloride lithium chloride in an anhydrous solvent such as tetrahydrofuran, followed by treatment with an electrophilic halogen source exemplified by but not limited to iodine. Imidazo[5,1-*f*][1,2,4]triazin-4(3*H*)-ones **I** are prepared from intermediate **II** by a cross-coupling reaction exemplified by but not limited to a Suzuki-Miyaura cross-coupling reaction. Such conditions for the cross coupling reaction are exemplified by but not limited to using; a boronic acid ester as the coupling partner, potassium phosphate as the base, a mixture of dimethylformamide and water as the solvent and a mixture of [1,1'-Bis(diphenylphosphino)ferrocene]dichloropalladium(II) (Pd(dppf)Cl₂) and Xantphos as the catalyst. In some examples R1 contains an unsaturated carbon-carbon bond which can be reduced by hydrogenation under conditions known to the person skilled in the art.

### Method 2:

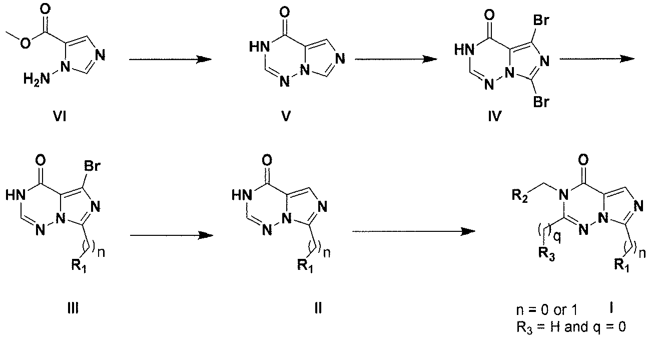

In brief, compounds **I** of the invention can be prepared from the commercially available methyl 1-amino-1*H*-imidazole-5-carboxylate (CAS 865444-80-0). Reaction of methyl 1-amino-1*H*-imidazole-5-carboxylate with formamidine acetate in a solvent such as ethanol gives imidazo[5,1-f][1,2,4]triazin-4(3*H*)-one **V.** Treatment of imidazo[5,1-f][1,2,4]triazin-4(3*H*)-one V with bromine in a solvent such as dimethylformamide yields 5,7-dibromoimidazo[5,1-*f*][1,2,4]]triazin-4(3*H*)-one **IV.** Intermediate **III** is prepared from intermediate **IV** by a cross-coupling reaction exemplified by but not limited to a Suzuki-Miyaura cross-coupling reaction. Such conditions for the cross coupling reaction are exemplified by but not limited to using: a boronic acid ester as the coupling partner, potassium carbonate as the base, a mixture of tetrahydrofuran and water as the solvent and Pd(dppf)Cl₂ as the catalyst. Intermediate **II** can be prepared from **III** by catalytic hydrogenation using a catalyst exemplified but not limited to palladium on carbon and an atmosphere of hydrogen in a solvent exemplified but not limited to a mixture of 1 M HCl (aq) and methanol. In the case where R1 contains an unsaturated carbon-carbon bond this is also reduced. Compounds **I** are prepared by alkylation of intermediate **II** using a suitable alkylating reagent exemplified but not limited to alkyl bromide in a suitable solvent exemplified by but not limited to dimethylformamide employing a base exemplified by but not limited to potassium carbonate.

### Method 3:

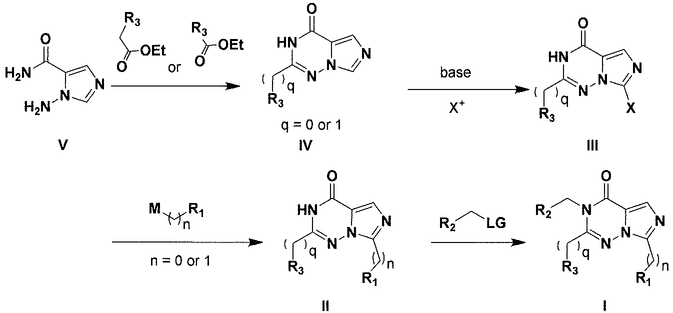

In brief, compounds **I** of the invention can be prepared from the commercially available 1-amino-1*H*-imidazole-5-carboxamide (CAS 1314910-72-9). Reacting 1-amino-1*H*-imidazole-5-carboxamide with a suitable acid derivative exemplified by but not limited to a carboxylic acid ester in a solvent, such as ethanol employing a base exemplified by but not limited to sodium ethoxide yields intermediate **IV.** Imidazo[5,1-*f*][1,2,4]triazin-4(3*H*)-ones **III** are prepared by treatment of **IV** by a strong base, exemplified by but not limited to 2,2,6,6-tetramethylpiperidinylmagnesium chloride lithium chloride in an anhydrous solvent such as tetrahydrofuran, followed by treatment with an electrophilic halogen source exemplified by but not limited to iodine. Imidazo[5,1-*f*][1,2,4]triazin-4(3*H*)-ones **II** are prepared from intermediate **III** by a cross coupling reaction exemplified by but not limited to a Suzuki-Miyaura cross-coupling reaction. Such conditions for the cross coupling reaction are exemplified by but not limited to using: a boronic acid ester as the coupling partner, potassium phosphate as the base, a mixture of dimethylformamide and water as the solvent and a mixture of Pd(dppf)Cl₂ and Xantphos as the catalyst. In some examples R1 contains an unsaturated carbon-carbon bond which can be reduced by hydrogenation under conditions known to the person skilled in the art. Compounds **I** are prepared by alkylation of intermediate **II** using a suitable alkylating reagent exemplified but not limited to alkyl iodide in a suitable solvent exemplified by but not limited to dimethylformamide employing a base exemplified by but not limited to potassium carbonate.

### General Methods

Analytical LC-MS data were obtained using the methods identified below.
Method 1: A Shimadzu 20 MS instrument equipped with atmospheric pressure photo ionisation ion source and a Shimadzu LC-20AB system was used. Column: MERCK, RP-18e 25-2mm; Column temperature: 50 °C; Solvent system: A = water/trifluoroacetic acid (99.9625.0375) and B = acetonitrile /trifluoroacetic acid (99.981:0.019); Method: A linear gradient elution A:B = 95:5 to A:B=5:95 in 0.7 minutes, then A:B=5:95 for 0.4 minutes, then with a linear gradient elution to A:B 95:5 for 0.4 minutes with a constant flow rate of 1.5 mL/min.
Method 2: A Shimadzu 20 MS instrument equipped with atmospheric pressure photo ionization ion source and a Shimadzu LC-20AB system was used. Column: Xtimate C18 2.1×30mm,3um; Column temperature: 50 °C; Solvent system: A = water/trifluoroacetic acid (99.9625.0375) and B = acetonitrile /trifluoroacetic acid 99.981:0.019); Method: A linear gradient elution A:B = 100:0 to A:B=70:30 in 0.9 minutes, then A:B=70:30 for 0.6 minutes, then with a linear gradient to A:B 0:100 for 0.5 minutes with a constant flow rate of 1.2 mL/min.
Method 3: An Agilent 1200 LCMS system with ELS detector was used. Column: Agilent TC-C18 5 µm ; 2.1x50mm; Column temperature: 50 °C; Solvent system: A = water/trifluoroacetic acid (99.9:0.1) and B = acetonitrile /trifluoroacetic acid (99.95:0.05); Method: Linear gradient elution with A:B = 99:1 to 0:100 in 4.0 minutes and with a flow rate of 0.8 mL/min.
Method 4: An Agilent 1200 LCMS system with ELS detector was used. Column: XBridge ShieldRP18, 5 µm, 50x2.1mm; Column temperature: 40 °C; Solvent system: A = water/NH₃^{∗}H₂O (99.95:0.05) and B = acetonitrile; Method: Linear gradient elution with A:B = 95:5 to 0:100 in 3.4 minutes and with a flow rate of 0.8 mL/min.
Method 5: An Agilent 1200 LCMS system with ELS detector was used. Column: Agilent TC-C18 5 µm; 2.1x50mm; Column temperature: 50 °C; Solvent system: A = water/trifluoroacetic acid (99.9:0.1) and B = acetonitrile /trifluoroacetic acid (99.95:0.05); Method: Linear gradient elution with A:B = 90:10 to 0:100 in 4.0 minutes and with a flow rate of 0.8 mL/min.

Preparative LC-MS-purification was performed on a PE Sciex API 150EX instrument with atmospheric pressure chemical ionization. Column: 50 X 20 mm YMC ODS-A with 5 µm particle size; Solvent system: A = water/trifluoroacetic acid (99.965:0.035) and B = acetonitrile /water/trifluoroacetic acid (94.965:5:0.035); Method: Linear gradient elution with A:B = 80:20 to 0:100 in 7 minutes and with a flow rate of 22.7 mL/minute. Fraction collection was performed by split-flow MS detection.

Preparative SFC was performed on a Thar 80 instrument. Exemplified conditions can be, but not limited to: Column AD 250 X 30mm with 20 µm particle size; Column temperature: 38 °C, Mobile phase: Supercritical CO₂/ EtOH (0.2%NH₃H₂O) =45/55.

### INTERMEDIATES

### Methyl 1-(di-(tert-butoxycarbonyl)amino)-1H-imidazole-5-carboxylate:

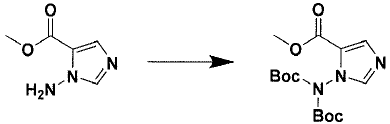

To a solution of methyl 1-amino-1*H*-imidazole-5-carboxylate (CAS 865444-80-0) (11.4 g, 80.8 mmol) in anhydrous dichloromethane (200 mL) was added *N,N*-dimethylpyridin-4-amine (4.93 g, 40.4 mmol) and triethylamine (12.3 g, 121 mmol), followed by di-*tert*-butyl dicarbonate (37 g, 0.17 mol). The reaction was stirred at room temperature for 2 hrs. The mixture was filtered, the filtrate was washed twice with 10% citric acid (aq) and then twice with sat NaHCO₃(aq) and finally with brine. The organic layer was dried over Na₂SO₄ and concentrated *in vacuo* to give methyl 1-(di-(*tert*-butoxycarbonyl)amino)-1*H*-imidazole-5-carboxylate 19 g (67%).

### Tert-butyl (5-((cyclohexylmethyl)carbamoyl)-1H-imidazol-1-yl)carbamate:

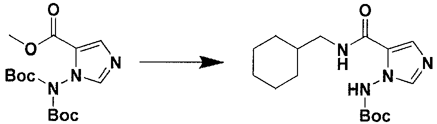

To a solution of methyl 1-(di-(*tert*-butoxycarbonyl)amino)-1*H*-imidazole-5-carboxylate (19 g, 56 mmol) and cyclohexylmethanamine (12.6 g, 111 mmol) in dry methanol (200 mL) was added K₂CO₃ (23.1 g, 167 mmol). The mixture was stirred at 70°C for 16 hrs. Then the mixture was concentrated *in vacuo* and diluted with dichloromethane (300 mL). The solution was washed with water (150 mL), then with brine (150 mL), dried over Na₂SO₄ and concentrated *in vacuo* to give *tert*-butyl (5-((cyclohexylmethyl)carbamoyl)-1*H*-imidazol-1-yl)carbamate 18 g (97%) used for next step directly.
LC-MS: (*m*/*z)* 323.1 (MH⁺) t_{R} (minutes, method 1) = 0.665 minutes

### 1-Amino-N-(cyclohexylmethyl)-1H-imidazole-5-carboxamide:

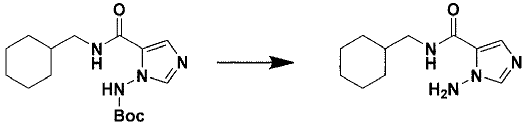

To a solution of *tert*-butyl (5-((cyclohexylmethyl)carbamoyl)-1*H*-imidazol-1-yl)carbamate (18 g, 55.8 mmol) in THF (200 mL) was added trifluoroacetic acid (20 mL). The solution was stirred at 60°C for 2 hrs and then cooled on an ice-bath and pH to pH 8-9 with sat. aq. NaHCO₃. The crude mixture was extracted with ethyl acetate (3x200 mL). The combined organic phases were washed with brine, dried over MgSO₄ and concentrated *in vacuo* to give 1-amino-*N*-(cyclohexylmethyl)-1*H*-imidazole-5-carboxamide 12 g (97%) that was used directly for the next step.
LC-MS: (*m*/*z)* 222.9 (MH⁺) t_{R} (minutes, method 1) = 0.561 minutes

### 3-(Cyclohexylmethyl)-2-methylimidazo[5,1-f][1,2,4]triazin-4(3H)-one:

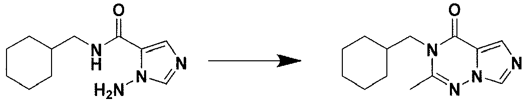

A suspension of 1-amino-*N*-(cyclohexylmethyl)-1*H*-imidazole-5-carboxamide (4.0 g, 18 mmol) in 1,1,1-triethoxyethane (10 mL) was stirred at 100°C for 16 hrs. Then Cs₂CO₃ (11.7 g, 36 mmol) was added and the mixture was stirred at 130°C for 3 hrs. The reaction was diluted with water (50 mL) and extracted with ethyl acetate (2x5O mL). The combined organic phases were washed with brine, dried over Na₂SO₄ and concentrated *in vacuo.*

The residue was purified by flash chromatography using a gradient of petroleum ether and ethyl acetate to afford 3-(cyclohexylmethyl)-2-methylimidazo[5,1-*f*][1,2,4]triazin-4(3*H*)-one 1.3 g (29%).
LC-MS: (*m*/*z)* 246.9 (MH⁺) t_{R} (minutes, method 1) = 0.672 minutes

### 3-(Cyclohexylmethyl)-7-iodo-2-methylimidazo[5,1-f][1,2,4]triazin-4(3H)-one:

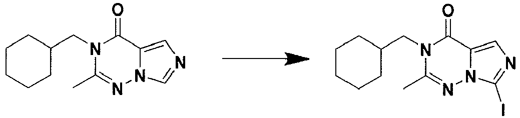

A solution of 3-(cyclohexylmethyl)-2-methylimidazo[5,1-*f*][1,2,4]triazin-4(3*H*)-one (400 mg, 1.62 mmol) in anhydrous THF (10 mL) was cooled to -40°C. A 1 M solution of 2,2,6,6-tetramethylpiperidinylmagnesium chloride lithium chloride in tetrahydrofuran/toluene (1 M, 3.25 mL) was added dropwise and the solution was stirred at -40°C for 1 hr. Then a solution of iodine (1.24 g, 4.87 mmol) in anhydrous THF (5 mL) was added drop-wise and the mixture was stirred at -40°C for 2 hrs. The reaction was quenched with Na₂S₂O₃ (aq), and extracted with ethyl acetate (2×30 mL). The combined organic phases were washed with brine, dried over Na₂SO₄ and concentrated *in vacuo.* The residue was purified by flash chromatography using a gradient of petroleum ether and ethyl acetate to afford 3-(cyclohexylmethyl)-7-iodo-2-methylimidazo[5,1-*f*][1,2,4]triazin-4(3*H*)-one 500 mg (83%).
LC-MS: (*m*/*z)* 372.9 (MH⁺) t_{R} (minutes, method 1) = 0.878 minutes

### 3-(Cyclohexylmethyl)-7-(3,6-dihydro-2H-pyran-4-yl)-2-methylimidazo[5,1-f][1,2,4]triazin-4(3H)-one:

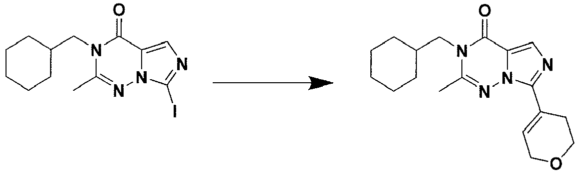

To a suspension of 3-(cyclohexylmethyl)-7-iodo-2-methylimidazo[5,1-*f*][1,2,4]triazin-4(3*H*)-one (450 mg, 1.21 mmol) and 2-(3,6-dihydro-2*H*-pyran-4-yl)-4,4,5,5-tetramethyl-1,3,2-dioxaborolane (762 mg, 3.63 mmol) in DMF (10 mL) and H₂O (3 mL) was added K₃PO₄ (770 mg, 3.63 mmol), Pd(dppf)Cl₂ (133 mg, 0.181 mmol) and Xantphos (210 mg, 0.363 mmol). The suspension was heated under microwave irradiation at 150°C for 1 hr. The mixture was filtered and the filtrate was diluted with water (20 mL), extracted with ethyl acetate (2×30 mL). The combined organic phases were washed with brine, dried over Na₂SO₄ and concentrated *in vacuo.* The residue was purified by flash chromatography using a gradient of petroleum ether and ethyl acetate to afford 3-(cyclohexylmethyl)-7-(3,6-dihydro-2*H*-pyran-4-yl)-2-methylimidazo[5,1-*f*][1,2,4]triazin-4(3*H*)-one 300 mg (75%).
LC-MS: (*m*/*z)* 328.9 (MH⁺) t_{R} (minutes, method 1) = 0.724 minutes

### 2-(4-Methylbenzyl)imidazo[5,1-f][1,2,4]triazin-4(3H)-one:

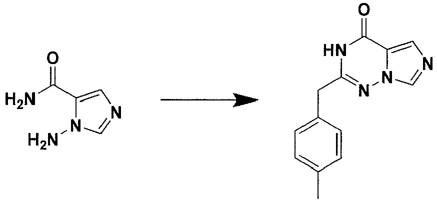

To a suspension of 1-amino-1*H*-imidazole-5-carboxamide (CAS: 1314910-72-9) (1.0 g, 7.9 mmol) in anhydrous ethanol (15 mL) was added sodium ethoxide (1.6 g, 24 mmol) and ethyl 2-(p-tolyl)acetate (4.2 g, 24 mmol). The mixture was heated under microwave irradiation at 140 °C for 1.5 hrs. The reaction was concentrated *in vacuo* and ice water (20 mL) was added before the pH was adjusted to 7 with 1M HCl (aq.). The suspension was filtered and the isolated solid was dried to afford 2-(4-methylbenzyl)imidazo[5,1-*f*][1,2,4]triazin-4(3*H*)-one 1.4 g (73%).

### 7-Iodo-2-(4-methylbenzyl)imidazo[5,1-f][1,2,4]triazin-4(3H)-one:

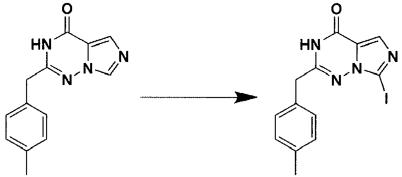

To a suspension of 2-(4-methylbenzyl)imidazo[5,1-*f*][1,2,4]triazin-4(3*H*)-one (5.9 g, 25 mmol) in anhydrous THF (55 mL) was added drop-wise a 1 M solution of 2,2,6,6-tetramethylpiperidinylmagnesium chloride lithium chloride in THF/toluene (73.7 mL) at -40°C under N₂ atmosphere. The mixture was stirred at -40°C for 1 hr and then a solution of iodine (6.2 g, 25 mmol) in anhydrous THF (5 mL) was added slowly at -40°C. The reaction was then stirred at -40°C for 1 hr. The reaction was quenched with aq. Na₂SO₃ (100 mL) and extracted with ethyl acetate (2×100 mL). The combined organic phases were washed with brine (50 mL), dried over Na₂SO₄ and concentrated *in vacuo.* The crude product was purified by flash chromatography using a gradient of petroleum ether and ethyl acetate to afford 7-iodo-2-(4-methylbenzyl)imidazo[5,1-*f*][1,2,4]triazin-4(3*H*)-one 3 g (33%).
LC-MS: (*m*/*z)* 366.7 (MH⁺) t_{R} (minutes, method 1) = 0.687 minutes

### 7-(3,6-Dihydro-2H-pyran-4-yl)-2-(4-methylbenzyl)imidazo[5,1-f][1,2,4]triazin-4(3H)-one:

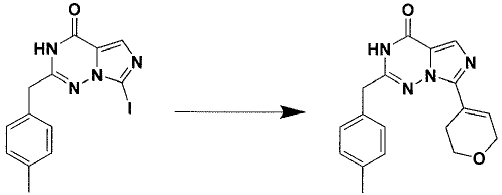

To a mixture of 7-iodo-2-(4-methylbenzyl)imidazo[5,1-*f*][1,2,4]triazin-4(3*H*)-one (2.5 g, 6.8 mmol) in DMF (10 mL) and H₂O (2 mL) was added 2-(3,6-dihydro-2*H*-pyran-4-yl)-4,4,5,5-tetramethyl-1,3,2-dioxaborolane (2.9 g, 14 mmol), K₃PO₄ (4.4 g, 20.5 mmol), Xantphos (1.2 g, 2.1 mmol) and Pd(dppf)Cl₂ (749 mg, 1.0 mmol). The mixture was heated under microwave irradiation at 140°C for 1 hr. The reaction mixture was diluted with ethyl acetate (200 mL), filtered and the filtrate was washed with water (2×50 mL), brine (50 mL), dried over Na₂SO₄ and concentrated *in vacuo.* The crude product was purified by flash chromatography using a gradient of petroleum ether and ethyl acetate to afford 7-(3,6-dihydro-2*H*-pyran-4-yl)-2-(4-methylbenzyl)imidazo[5,1-*f*][1,2,4]triazin-4(3*H*)-one 1.5 g (68%).
LC-MS: (*m*/*z)* 322.9 (MH⁺) t_{R} (minutes, method 1) = 0.787 minutes

### 2-(4-Methylbenzyl)-7-(tetrahydro-2H-pyran-4-yl)imidazo[5,1-f][1,2,4]triazin-4(3H)-one:

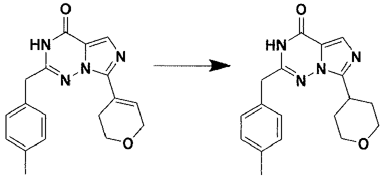

To a solution 7-(3,6-dihydro-2*H*-pyran-4-yl)-2-(4-methylbenzyl)imidazo[5,1-*f*][1,2,4]triazin-4(3*H*)-one (1.8 g, 5.6 mmol) in a mixture of methanol (50 mL) and ethyl acetate (50 mL) was added Pd/C (500 mg, wet, 10% of Pd with 50% of water). The reaction was stirred at 40°C under a balloon of H₂ for 5 hrs. The reaction mixture was filtered and the filtrate was concentrated *in vacuo* to afford 2-(4-methylbenzyl)-7-(tetrahydro-2*H*-pyran-4-yl)imidazo[5,1-*f*][1,2,4]triazin-4(3*H*)-one 1.5 g (83%).
LC-MS: (*m*/*z)* 324.9 (MH⁺) t_{R} (minutes, method 1) = 0.787 minutes

### 2-Methylimidazo[5,1-f][1,2,4]triazin-4(3H)-one:

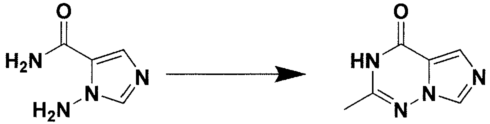

A solution of 1-amino-1*H*-imidazole-5-carboxamide (4.0 g, 32 mmol) in 1,1,1-triethoxyethane (100 mL) was heated at 100°C for 16 hrs. Then Cs₂CO₃ (20.7 g, 63.4 mmol) was added and the reaction was heated at 130°C for 3 hrs. The mixture was concentrated and purified by flash chromatography on silica gel using a gradient of dichloromethane and methanol to give 2-methylimidazo[5,1-*f*][1,2,4]triazin-4(3*H*)-one 2.0 g (42%).

### 7-Iodo-2-methylimidazo[5,1-f][1,2,4]triazin-4(3H)-one:

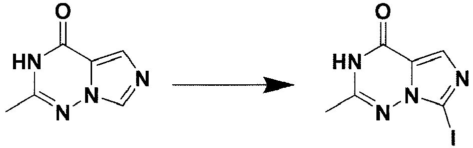

To a solution of 2-methylimidazo[5,1-*f*][1,2,4]triazin-4(3*H*)-one (2.0 g, 13 mmol) in tetrahydrofuran (20 mL) was drop-wise added a 1 M solution of 2,2,6,6-tetramethylpiperidinylmagnesium chloride lithium chloride in tetrahydrofuran/toluene (40 mL) at -40°C. The mixture was stirred at -40°C for 1 hr. Then a solution of iodine (10 g, 40 mmol) in tetrahydrofuran (20 mL) was added dropwise at -40°C. The mixture was stirred at-40°C for 1 hr and then quenched with aq. Na₂SO₃ (50 mL). The crude mixture was extracted with ethyl acetate (2×50 mL). The combined organic phases were washed with H₂O (50 mL), dried over Na₂SO₄, filtered, concentrated *in vacuo* and purified by flash chromatography using a gradient of ethyl acetate and methanol to give 7-iodo-2-methylimidazo[5,1-*f*][1,2,4]triazin-4(3*H*)-one 1.7 g (46%).
LC-MS: (*m*/*z)* 276.7 (MH⁺) t_{R} (minutes, method 2) = 0.961 minutes

### 7-(3,6-Dihydro-2H-pyran-4-yl)-2-methylimidazo[5,1-f][1,2,4]triazin-4(3H)-one:

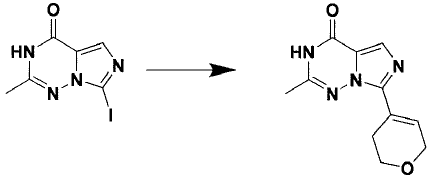

To a suspension of 7-iodo-2-methylimidazo[5,1-*f*][1,2,4]triazin-4(3*H*)-one (1.60 g, 5.80 mmol) and 2-(3,6-dihydro-2*H*-pyran-4-yl)-4,4,5,5-tetramethyl-1,3,2-dioxaborolane (1.83 g, 8.70 mmol) in dioxane (15 mL) and H₂O (5 mL) was added Pd(dppf)Cl₂ (848 mg, 1.16 mmol) and potassium carbonate (1.60 g, 11.6 mmol). The reaction mixture was degassed with N₂ and heated at 100°C for 16 hrs. The crude reaction mixture was concentrated and purified by flash chromatography using a gradient of dichloromethane and methanol to give 7-(3,6-dihydro-2*H*-pyran-4-yl)-2-methylimidazo[5,1-*f*][1,2,4]triazin-4(3*H*)-one 1.1 g (82%).

### 2-Methyl-7-(tetrahydro-2H-pyran-4-yl)imidazo[5,1-f][1,2,4]triazin-4(3H)-one:

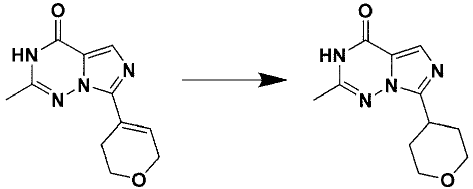

To a solution of 7-(3,6-dihydro-2*H*-pyran-4-yl)-2-methylimidazo[5,1-*f*][1,2,4]triazin-4(3*H*)-one (1.0 g, 4.3 mmol) in methanol (50 mL) was added Pd/C (500 mg, dry, 10% Pd). The mixture was stirred at 50°C under H₂ (15 psi) for 5 hrs. The mixture was filtered and the filtrate was concentrated to give 2-methyl-7-(tetrahydro-2*H*-pyran-4-yl)imidazo[5,1-*f*][1,2,4]triazin-4(3*H*)-one 800 mg (79%).

### 2-Ethylimidazo[5,1-f][1,2,4]triazin-4(3H)-one:

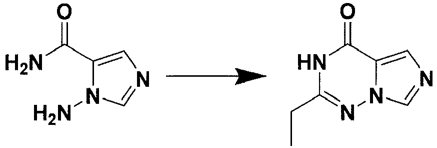

1-Amino-1*H*-imidazole-5-carboxamide (150 mg, 1.19 mmol), ethyl propionate (486 mg, 4.76 mmol, 4.0 eq) and sodium ethoxide (324 mg, 4.76 mmol) were suspended in ethanol (5 mL) and heated under microwave irradiation for 1 hr at 140 °C. The reaction mixture was concentrated *in vacuo* and purified by flash chromatography using a gradient of dichloromethane and methanol to give 2-ethylimidazo[5,1-*f*][1,2,4]triazin-4(3*H*)-one 82 mg (42%).

### 2-Ethyl-7-iodoimidazo[5,1-f][1,2,4]triazin-4(3H)-one:

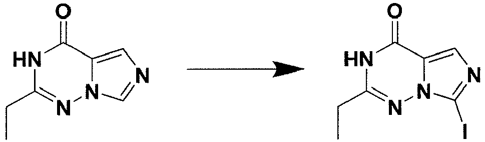

To a solution of 2-ethylimidazo[5,1-*f*][1,2,4]triazin-4(3*H*)-one (250 mg, 1.52 mmol) in dry tetrahydrofuran (10 mL) was added a 1 M solution of 2,2,6,6-tetramethylpiperidinylmagnesium chloride lithium chloride in tetrahydrofuran/toluene (4.56 mL) dropwise at -40°C. The reaction was stirred at -40°C for 1 hour before addition of iodine (1.16 g, 4.56 mmol, 3.0 eq) in dry tetrahydrofuran (10 mL) at -40°C. After stirring another 1 hr at -40°C the reaction was quenched by addition of sat. NH₄Cl (aq) (30 mL). The crude reaction was and extracted with ethyl acetate (3×30 mL). The combined organic phases were washed with saturated sodium sulfite (20 mL), dried over anhydrous Na₂SO₄ and concentrated *in vacuo.* The residue was purified by flash chromatography using a gradient of dichloromethane and methanol to afford 2-ethyl-7-iodoimidazol[5,1-*f*][1,2,4]triazin-4(3*H*)-one 410 mg (93%).

### 7-(3,6-Dihydro-2H-pyran-4-yl)-2-ethylimidazo[5,1-f][1,2,4]triazin-4(3H)-one:

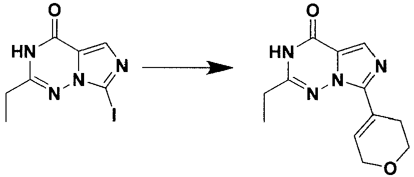

To a mixture of 2-ethyl-7-iodoimidazo[5,1-*f*][1,2,4]triazin-4(3*H*)-one (400 mg, 1.38 mmol) and 2-(3,6-dihydro-2*H*-pyran-4-yl)-4,4,5,5-tetramethyl-1,3,2-dioxaborolane (435 mg, 2.07 mmol) in dioxane (10 mL) and H₂O (0.100 mL), were added Pd(dppf)Cl₂.CH₂Cl₂ (113 mg, 0.138 mmol) and Na₂CO₃ (293 mg, 2.76 mmol) at 20°C under N₂. The mixture was stirred at 100°C for 16 hrs. The reaction solution was concentrated *in vacuo* and adjusted to pH 8∼9 with saturated NaHCO₃ (20 mL). The crude reaction was extracted with ethyl acetate (3x20 mL), dried over anhydrous sodium sulfate, and concentrated *in vacuo.* The residue was purified by flash chromatography using dichloromethane and methanol to give 7-(3,6-dihydro-2*H-*pyran-4-yl)-2-ethylimidazo[5,1-*f*][1,2,4]triazin-4(3*H*)-one 270 mg (79%).

### 2-Ethyl-7-(tetrahydro-2H-pyran-4-yl)imidazo[5,1-f][1,2,4]triazin-4(3H)-one:

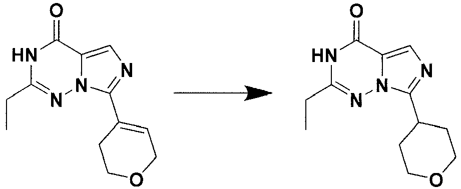

To a solution of 7-(3,6-dihydro-2*H*-pyran-4-yl)-2-ethylimidazo[5,1-*f*][1,2,4]triazin-4(3*H*)-one (270 mg, 1.1 mmol) in methanol (30 mL) was added Pd/C (100 mg, 10% Pd) under N₂. The suspension was degassed under vacuum and purged with H₂ several times. The mixture was stirred under H₂ (15 psi) at 25°C for 5 hrs. The reaction mixture was filtered and the filtrate was concentrated *in vacuo.* The residue was purified by flash chromatography using a gradient of dichloromethane and methanol to give 2-ethyl-7-(tetrahydro-2*H*-pyran-4-yl)imidazo[5,1-*f*][1,2,4]triazin-4(3*H*)-one 110 mg (40%).

### Imidazo[5,1-f][1,2,4]triazin-4(3H)-one:

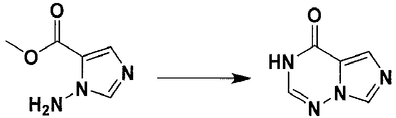

To a suspension of methyl 1-amino-1*H*-imidazole-5-carboxylate (5.00 g, 35.4 mmol) in anhydrous ethanol (50 mL) was added formamidine acetate (11.1 g, 106 mmol). The mixture was stirred keeping the temperature at 80-90°C for 16 hrs. The reaction was cooled and the solid was collected by filtration, washed with ethanol (2×30 mL) and petroleum ether (2×30 mL). The product was then dried *in vacuo* to afford imidazo[5,1-*f*][1,2,4]triazin-4(3*H*)-one 4.8 g (99%).

### 5,7-Dibromoimidazo[5,1-f][1,2,4]triazin-4(3H)-one:

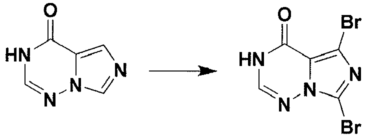

To a solution of imidazo[5,1-*f*][1,2,4]triazin-4(3*H*)-one (4.8 g, 35.3 mmol) in dimethylformamide (40 mL) was added bromine (16.9 g, 106 mmol) at 0°C. The reaction was stirred at 0°C for 4 hrs. The reaction mixture was quenched with aq. Na₂SO₃ (150 mL) and the suspension was filtered, the filter cake was dried to afford 5,7-dibromoimidazo[5,1-*f*][1,2,4]triazin-4(3*H*)-one 9 g (87%).

### 5-Bromo-7-(3,6-dihydro-2H-pyran-4-yl)imidazo[5,1-f][1,2,4]triazin-4(3H)-one:

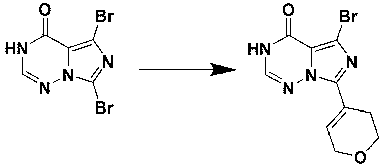

To a suspension of 5,7-dibromoimidazo[5,1-*f*][1,2,4]triazin-4(3*H*)-one (1.0 g, 3.4 mmol) in tetrahydrofuran (10 mL) and H₂O (3 mL) was added 2-(3,6-dihydro-2*H*-pyran-4-yl)-4,4,5,5-tetramethyl-1,3,2-dioxaborolane (857 mg, 4.07 mmol), Pd(dppf)Cl₂ (498 mg, 0.68 mmol), potassium carbonate (564 mg, 4.1 mmol). The mixture was heated under microwave irradiation at 110°C for 2 hrs. A 1 M aqueous solution of HCI (50 mL) was added, the mixture was extracted with ethyl acetate (3×50 mL). The combined organic phases were washed with brine (20 mL) and dried over Na₂SO₄. The crude product was purified by flash chromatography using a gradient of dichloromethane and methanol to afford 5-bromo-7-(3,6-dihydro-2*H*-pyran-4-yl)imidazo[5,1-*f*][1,2,4]triazin-4(3*H*)-one 250 mg (20%).

### 7-(Tetrahydro-2H-pyran-4-yl)imidazo[5,1-f][1,2,4]triazin-4(3H)-one:

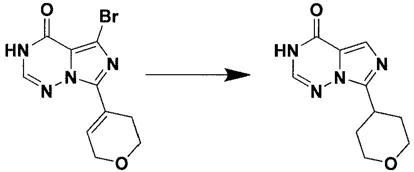

To a solution of 5-bromo-7-(3,6-dihydro-2*H*-pyran-4-yl)imidazo[5,1-*f*][1,2,4]triazin-4(3*H*)-one (400 mg, 1.4 mmol) in methanol (20 mL) and 1 M aqueous HCl (10 mL) was added Pd/C (200 mg, wet, 10% of Pd with 50% of water). The mixture was stirred at room temperature for 5 hrs under a balloon of H₂. The mixture was filtered and the flitrate was concentrated *in vacuo.* The residue was purified by preparative HPLC to afford 7-(tetrahydro-2*H*-pyran-4-yl)imidazo[5,1-*f*][1,2,4]triazin-4(3*H*)-one 35 mg (12%).
¹H NMR (DMSO-d6, 400 MHz): 7.91 (s, 1H), 7.70 (s, 1H), 3.95-3.92 (m, 2H), 3.51-3.45 (m, 2H), 3.39-3.34 (m, 1H), 1.87-1.81 (m, 4H).
LC-MS: (*m*/*z)* 221.1 (MH⁺) t_{R} (minutes, method 3) = 1.60 minutes

### COMPOUNDS OF THE INVENTION:

### Example 1:

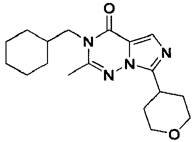

### 3-(Cyclohexylmethyl)-2-methyl-7-(tetrahydro-2H-pyran-4-yl)imidazo[5,1-f][1,2,4]triazin-4(3H)-one:

To a solution of 3-(cyclohexylmethyl)-7-(3,6-dihydro-2*H*-pyran-4-yl)-2-methylimidazo[5,1-*f*][1,2,4]triazin-4(3*H*)-one (250 mg, 0.761 mmol) in methanol (5 mL) was added Pd/C (wet, 100 mg, 10% Pd with 50% of water). The mixture was stirred at room temperature under a balloon of H₂ for 1 hr. The crude mixture was filtered and the filtrate was concentrated *in vacuo.* The residue was purified by preparative HPLC to afford 3-(cyclohexylmethyl)-2-methyl-7-(tetrahydro-2*H*-pyran-4-yl)imidazo[5,1-*f*][1,2,4]triazin-4(3*H*)-one 120 mg (48%).
¹H NMR (DMSO-d6, 400 MHz): *δ* 7.67 (s, 1H), 3.95-3.91 (m, 2H), 3.78 (d, *J*=7.6 Hz, 2H), 3.49-3.35 (m, 3H), 2.47 (s, 3H), 1.84-1.57 (m, 10H), 1.15-0.99 (m, 5H).
LC-MS: (*m*/*z)* 331.2 (MH⁺) t_{R} (minutes, method 3) = 2.76 minutes

### Example 2:

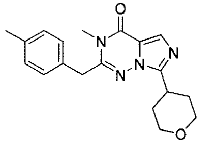

### 3-Methyl-2-(4-methylbenzyl)-7-(tetrahydro-2H-pyran-4-yl)imidazo[5,1-f][1,2,4]triazin-4(3H)-one:

To a solution of 2-(4-methylbenzyl)-7-(tetrahydro-2*H*-pyran-4-yl)imidazo[5,1-*f*][1,2,4]triazin-4(3*H*)-one (70 mg, 0.22 mmol) in anhydrous DMF (3 mL) was added K₂CO₃ (89 mg, 0.65 mmol) and iodomethane (370 mg, 2.60 mmol). The mixture was stirred at 40°C for 2 hrs. The reaction mixture was cooled to room temperature and filtered. The filtrate was purified by preparative HPLC to afford 3-methyl-2-(4-methylbenzyl)-7-(tetrahydro-2*H*-pyran-4-yl)imidazo[5,1-*f*][1,2,4]triazin-4(3*H*)-one 30 mg (41%).
¹H NMR (CDCl₃, 400): *δ*7.83 (s, 1H), 7.20-7.14 (m, 4H), 4.14-4.08 (m, 4H), 3.65-3.59 (m, 2H), 3.49-3.46 (m, 1H), 3.36 (s, 3H), 2.37 (s, 3H), 2.19-2.09 (m, 2H), 2.00-1.97 (m, 2H).
LC-MS: (*m*/*z)* 339.2 (MH⁺) t_{R} (minutes, method 4) = 2.24 minutes

### Example 3:

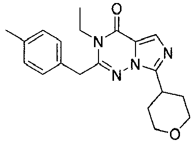

### 3-Ethyl-2-(4-methylbenzyl)-7-(tetrahydro-2H-pyran-4-yl)imidazo[5,1-f][1,2,4]triazin-4(3H)-one:

To a solution of 2-(4-methylbenzyl)-7-(tetrahydro-2*H*-pyran-4-yl)imidazo[5,1-*f*][1,2,4]triazin-4(3*H*)-one (70 mg, 0.22 mmol) in anhydrous DMF (3 mL) was added K₂CO₃ (89 mg, 0.65 mmol) and iodoethane (67 mg, 0.43 mmol). The mixture was stirred at 50°C for 1.5 hrs. The reaction mixture was filtered and the filtrate was purified by preparative HPLC to afford 3-ethyl-2-(4-methylbenzyl)-7-(tetrahydro-2*H*-pyran-4-yl)imidazo[5,1-*f*][1,2,4]triazin-4(3*H*)-one 40 mg (52%).
¹H NMR (CDCl₃, 400 MHz): *δ* 7.82 (s, 1H), 7.21-7.15 (m, 4H), 4.13-4.11 (m, 2H), 4.06 (s, 2H), 3.91 (q, *J* = 7.2 Hz, 2H), 3.64-3.58 (m, 2H), 3.48-3.46 (m, 1H), 2.37 (s, 3H), 2.19-2.10 (m, 2H), 2.08-1.96 (m, 2H), 1.16 (t, *J* = 7.2 Hz, 3H).
LC-MS: (*m*/*z*) 353.2 (MH⁺) t_{R} (minutes, method 3) = 2.82 minutes

### Example 4:

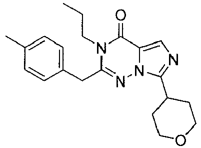

### 2-(4-Methylbenzyl)-7-(tetrahydro-2H-pyran-4-yl)imidazo[5,1-f][1,2,4]triazin-4(3H)-one:

To a solution of 2-(4-methylbenzyl)-7-(tetrahydro-2*H*-pyran-4-yl)imidazo[5,1-*f*][1,2,4]triazin-4(3*H*)-one (70 mg, 0.22 mmol) in anhydrous DMF (3 mL) was added K₂CO₃ (90 mg, 0.65 mmol) and 1-bromopropane (53 mg, 0.43 mmol). The mixture was stirred at 50°C for 1.5 hrs. The mixture was cooled to room temperature and filtered, the filtrate was purified by preparative HPLC to afford 2-(4-methylbenzyl)-3-propyl-7-(tetrahydro-2*H*-pyran-4-yl)imidazo[5,1-*f*][1,2,4]triazin-4(3*H*)-one 15 mg (18%).
¹H NMR (CDCl₃, 400 MHz): *δ* 7.78 (s, 1H), 7.17-7.10 (m, 4H), 4.09-4.07 (m, 2H), 4.01 (s, 2H), 3.75 (t, *J* = 7.6 Hz, 2H), 3.60-3.55 (m, 2H), 3.46-3.42 (m, 1H), 2.54 (s, 3H), 2.15-2.05 (m, 2H), 1.95-1.92 (m, 2H), 1.55-1.49 (m, 2H), 0.88 (t, *J* = 7.2 Hz, 3H).
LC-MS: (*m*/*z)* 367.2 (MH⁺) t_{R} (minutes, method 3) = 2.98 minutes

### Example 5:

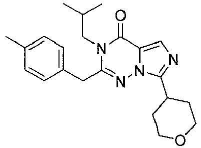

### 3-Isobutyl-2-(4-methylbenzyl)-7-(tetrahydro-2H-pyran-4-yl)imidazo[5,1-f][1,2,7]triazin-4(3H)-one:

To a solution of 2-(4-methylbenzyl)-7-(tetrahydro-2*H*-pyran-4-yl)imidazo[5,1-*f*][1,2,4]triazin-4(3*H*)-one (70 mg, 0.22 mmol) in anhydrous DMF (3 mL) was added K₂CO₃ (90 mg, 0.65 mmol) and 1-bromo-2-methylpropane (59 mg, 0.43 mmol). The mixture was stirred at 65°C for 16 hrs. The reaction mixture was filtered and the filtrate was purified by preparative HPLC to afford 3-isobutyl-2-(4-methylbenzyl)-7-(tetrahydro-2*H*-pyran-4-yl)imidazo[5,1-*f*][1,2,4]triazin-4(3*H*)-one 20 mg (23%).
¹H NMR (CDCl₃, 400 MHz): *δ* 7.78 (s, 1H), 7.17-7.08 (m, 4H), 4.10-4.07 (m, 2H), 4.03 (s, 2H), 3.68 (d, *J* = 7.2 Hz, 2H), 3.60-3.55 (m, 2H), 3.46-3.39 (m, 1H), 2.39 (s, 3H), 2.14-2.05 (m, 3H), 1.96-1.93 (m, 2H), 0.94 (d, *J* = 6.8 Hz, 6H).
LC-MS: (*m*/*z)* 381.2 (MH⁺) t_{R} (minutes, method 3) = 3.10 minutes

### Example 6:

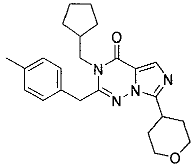

### 3-(Cyclopentylmethyl)-2-(4-methylbenzyl)-7-(tetrahydro-2H-pyran-4-yl)imidazo[5,1-f][1,2,4]triazin-4(3H)-one:

A solution of 2-(4-methylbenzyl)-7-(tetrahydro-2*H*-pyran-4-yl)imidazo[5,1-*f*][1,2,4]triazin-4(3*H*)-one (100 mg, 0.308 mmol), bromomethylcyclopentane (75 mg, 0.46 mmol) and K₂CO₃ (85 mg, 0.62 mmol) in DMF (4 mL) was stirred at 65°C for 16 hrs. The mixture was filtered and the filtrate was purified by preparative HPLC to yield 3-(cyclopentylmethyl)-2-(4-methylbenzyl)-7-(tetrahydro-2*H*-pyran-4-yl)imidazo[5,1-*f*][1,2,4]triazin-4(3*H*)-one 20 mg (16%).
¹H NMR (CDCl₃, 400 MHz): *δ* 7.77 (s, 1H), 7.15 (d, *J* = 8.0 Hz, 2H), 7.08 (d, *J* = 8.0 Hz, 2H), 4.09-4.05 (m, 4H), 3.81 (d, *J* = 7.2 Hz, 2H), 3.60-3.54 (m, 2H), 3.42-3.39 (m, 1H), 2.33 (s, 3H), 2.18-2.07 (m, 3H), 1.95-1.92 (m, 2H), 1.69-1.68 (m, 4H), 1.55-1.53 (m, 2H), 1.28-1.25 (m, 2H).
LC-MS: (*m*/*z)* 407.5 (MH⁺) t_{R} (minutes, Method 3) = 3.28 minutes

### Example 7:

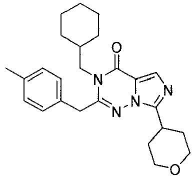

### 3-(Cyclohexylmethyl)-2-(4-methylbenzyl)-7-(tetrahydro-2H-pyran-4-yl)imidazo[5,1-f][1,2,4]triazin-4(3H)-one:

A solution of 2-(4-methylbenzyl)-7-(tetrahydro-2*H*-pyran-4-yl)imidazo[5,1-*f*][1,2,4]triazin-4(3*H*)-one (100 mg, 0.308 mmol), bromomethylcyclohexane (109 mg, 0.617 mmol) and K₂CO₃ (128 mg, 0.925 mmol) in DMF (4 mL) was stirred at 50°C for 2 hrs. Then the mixture was heated to 65°C and stirred for 16 hrs. The mixture was filtered and the filtrate was purified by preparative HPLC to give 3-(cyclohexylmethyl)-2-(4-methylbenzyl)-7-(tetrahydro-2*H*-pyran-4-yl)imidazo[5,1-*f*][1,2,4]triazin-4(3*H*)-one 12 mg (9%).
¹H NMR (CD₃OD, 400 MHz): *δ* 7.69 (s, 1H), 7.17 (s, 4H), 4.13 (s, 2H), 4.03-3.99 (m, 2H), 3.75-3.73 (m, 2H), 3.57-3.51 (m, 3H), 2.31 (s, 3H), 1.99-1.56 (m, 5H), 1.85-1.60 (m, 5H), 1.15 (brs, 3H), 1.01-0.99 (m, 2H).
LC-MS: (*m*/*z)* 421.6 (MH⁺) t_{R} (minutes, Method 5) = 2.88 minutes

### Example 8:

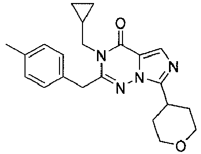

### 3-(Cyclopropylmethyl)-2-(4-methylbenzyl)-7-(tetrahydro-2H-pyran-4-yl)imidazo[5,1-f][1,2,4]triazin-4(3H)-one:

A solution of 2-(4-methylbenzyl)-7-(tetrahydro-2*H*-pyran-4-yl)imidazo[5,1-*f*][1,2,4]triazin-4(3*H*)-one (100 mg, 0.310 mmol), bromomethylcyclopropane (62 mg, 0.46 mmol) and K₂CO₃ (85 mg, 0.62 mmol) in DMF (4 mL) was stirred at 65°C for 16 hrs. The mixture was filtered and the filtrate was purified by preparative HPLC to give 3-(cyclopropylmethyl)-2-(4-methylbenzyl)-7-(tetrahydro-2*H*-pyran-4-yl)imidazo[5,1-*f*][1,2,4]triazin-4(3*H*)-one 23 mg (20%).
¹H NMR (CDCl₃, 400 MHz): *δ* 7.81 (s, 1H), 7.19-7.12 (m, 4H), 4.11 (s, 4H), 3.79 (d, *J* = 6.4 Hz, 2H), 3.62-3.56 (m, 2H), 3.47-3.42 (m, 1H), 2.36 (s, 3H), 2.17-2.08 (m, 2H), 2.07-1.95 (m, 2H), 1.03-0.97 (m, 1H), 0.57-0.53 (m, 2H), 0.44-0.42 (m, 2H).
LC-MS: (*m*/*z)* 379.5 (MH⁺) t_{R} (minutes, method 4) = 2.58 minutes

### Example 9:

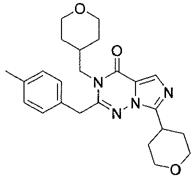

### 2-(4-Methylbenzyl)-7-(tetrahydro-2H-pyran-4-yl)-3-((tetrahydro-2H-pyran-4-yl)methyl)imidazo[5,1-f][1,2,4]triazin-4(3H)-one:

A mixture of 2-(4-methylbenzyl)-7-(tetrahydro-2*H*-pyran-4-yl)imidazo[5,1-*f*][1,2,4]triazin-4(3*H*)-one (100 mg, 0.308 mmol), 4-(bromomethyl)tetrahydropyran (83 mg, 0.46 mmol) and K₂CO₃ (85 mg, 0.62 mmol) in DMF (4 mL) was stirred at 65°C for 16 hrs. The mixture was filtered and the filtrate was purified by preparative HPLC to give 2-(4-methylbenzyl)-7-(tetrahydro-2*H*-pyran-4-yl)-3-((tetrahydro-2*H*-pyran-4-yl)methyl)imidazo[5,1-*f*][1,2,4]triazin-4(3*H*)-one 12 mg (9%).
¹H NMR (CDCl₃, 400 MHz): *δ* 7.78 (s, 1H), 7.16 (d, *J* = 8.0 Hz, 2H), 7.08 (d, *J* = 8.0 Hz, 2H), 4.10-4.04 (m, 4H), 3.95-3.93 (m, 2H), 3.73 (d, *J* = 6.8 Hz, 2H), 3.60-3.54 (m, 2H), 3.45-3.35 (m, 1H), 3.30-3.24 (m, 2H), 2.34 (s, 3H), 2.12-2.06 (m, 2H), 1.95-1.92 (m, 3H), 1.56-1.53 (m, 2H), 1.44-1.38 (m, 2H).
LC-MS: (*m*/*z)* 423.5 (MH⁺) t_{R} (minutes, method 3) = 2.85 minutes

### Example 10

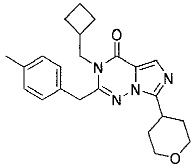

### 3-(Cyclobutylmethyl)-2-(4-methylbenzyl)-7-(tetrahydro-2H-pyran-4-yl)imidazo[5,1-f][1,2,4]triazin-4(3H)-one:

A solution of 2-(4-methylbenzyl)-7-(tetrahydro-2*H*-pyran-4-yl)imidazo[5,1-*f*][1,2,4]triazin-4(3*H*)-one (100 mg, 0.308 mmol), bromomethylcyclobutane (69 mg, 0.46 mmol.) and K₂CO₃ (85 mg, 0.62 mmol) in DMF (4 mL) was stirred at 65°C for 16 hrs. The mixture was filtered and the filtrate was purified by preparative HPLC to give 3-(cyclobutylmethyl)-2-(4-methylbenzyl)-7-(tetrahydro-2*H*-pyran-4-yl)imidazo[5,1-*f*][1,2,4]triazin-4(3*H*)-one 10 mg (8%).
¹H NMR (CDCl₃, 400 MHz): *δ* 7.71 (s, 1H), 7.10 (d, *J* = 8.0 Hz, 2H), 7.03 (d, *J* = 7.6 Hz, 2H), 4.03 (d, *J* = 12.0 Hz, 2H), 3.96 (s, 2H), 3.83 (d, *J* = 6.8 Hz, 2H), 3.54-3.48 (m, 2H), 3.37-3.35 (m, 1H), 2.52-2.49 (m, 1H), 2.28 (s, 3H), 2.05-1.86 (m, 6H), 1.76 (brs, 4H).
LC-MS: (*m*/*z)* 393.5 (MH⁺) t_{R} (minutes, method 3) = 3.15 minutes

### Example 11

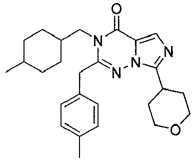

### 2-(4-Methylbenzyl)-3-((4-methylcyclohexyl)methyl)-7-(tetrahydro-2H-pyran-4-yl)imidazo[5,1-f][1,2,4]triazin-4(3H)-one:

A solution of 2-(4-methylbenzyl)-7-(tetrahydro-2*H*-pyran-4-yl)imidazo[5,1-*f*][1,2,4]triazin-4(3*H*)-one (280 mg, 0.902 mmol), (4-methylcyclohexyl)methyl methanesulfonate (CAS 272780-72-0) (560 mg) and K₂CO₃ (312 mg, 2.30 mmol) in DMF (10 mL) was stirred at 90°C for 16 hrs. The mixture was concentrated and the residue was purified by preparative HPLC to give 2-(4-methylbenzyl)-3-((4-methylcyclohexyl)methyl)-7-(tetrahydro-2*H*-pyran-4-yl)imidazo[5,1-*f*][1,2,4]triazin-4(3*H*)-one 37 mg (9%).

The mixture of cis- and trans-isomers of 2-(4-methylbenzyl)-3-((4-methylcyclohexyl)methyl)-7-(tetrahydro-2*H*-pyran-4-yl)imidazo[5,1-*f*][1,2,4]triazin-4(3*H*)-one (50 mg) was purified by SFC separation (column: AD(250mm^{∗}30mm, 5µm) and numbered according to the order of elution:
**Stereoisomer 1:** *cis*-2-(4-Methylbenzyl)-3-((4-methylcyclohexyl)methyl)-7-(tetrahydro-2*H-*pyran-4-yl)imidazo[5,1-*f*][1,2,4]triazin-4(3*H*)-one
   10 mg (20%)
   ¹H NMR (CD₃OD, 400 MHz): *δ* 7.75 (brs, 1H), 7.20-7.18 (m, 4H), 4.16 (s, 2H), 4.06-4.03 (m, 2H), 3.78 (d, *J* = 7.2 Hz, 2H), 3.61-3.48 (m, 3H), 2.35 (s, 3H), 2.06-1.91 (m, 4H), 1.71-1.51 (m, 5H), 1.34-1.32 (m, 1H), 1.12-1.02 (m, 2H), 0.88-0.82 (m, 5H).
   LC-MS: (*m*/*z)* 435.2 (MH⁺) t_{R} (minutes, method 5) = 2.97 minutes
**Stereoisomer 2:** *trans*-2-(4-Methylbenzyl)-3-((4-methylcyclohexyl)methyl)-7-(tetrahydro-2*H-*pyran-4-yl)imidazo[5,1-*f*][1,2,4]triazin-4(3*H*)-one
   10 mg (20%).
   ¹H NMR (CD₃OD, 400 MHz): δ 7.60 (s, 1H), 7.14-7.03 (m, 4H), 4.04 (s, 2H), 3.94-3.91 (m, 2H), 3.76 (d, *J* = 6.8 Hz, 2H), 3.49-3.34 (m, 3H), 2.23 (s, 3H), 1.94-1.79 (m, 4H), 1.70-1.55 (m, 2H), 1.38-1.21 (m, 8H), 0.84 (d, *J* = 6.4 Hz, 3H).
   LC-MS: (*m*/*z)* 435.2 (MH⁺) t_{R} (minutes, method 5) = 2.95 minutes

### Example 12

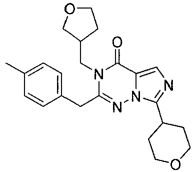

### 2-(4-Methylbenzyl)-7-(tetrahydro-2H-pyran-4-yl)-3-((tetrahydrofuran-3-yl)methyl)imidazo[5,1-f][1,2,4]triazin-4(3H)-one:

A mixture of 2-(4-methylbenzyl)-7-(tetrahydro-2*H*-pyran-4-yl)imidazo[5,1-*f*][1,2,4]triazin-4(3*H*)-one (200 mg, 0.617 mmol), (tetrahydrofuran-3-yl)methyl methanesulfonate(CAS 184849-49-8) (222 mg, 1.23 mmol) and K₂CO₃ (213 mg, 1.54 mmol) in DMF (5 mL) was stirred at 90°C for 16 hrs. The mixture was filtered and the filtrated was purified by preparative HPLC to give 2-(4-methylbenzyl)-7-(tetrahydro-2*H*-pyran-4-yl)-3-((tetrahydrofuran-3-yl)methyl)imidazo[5,1-*f*][1,2,4]triazin-4(3*H*)-one 65 mg (26%).

The racemic mixture of 2-(4-methylbenzyl)-7-(tetrahydro-2*H*-pyran-4-yl)-3-((tetrahydrofuran-3-yl)methyl)imidazo[5,1-*f*][1,2,4]triazin-4(3*H*)-one (65 mg) was purified by SFC separation (Column: AS(250mm^{∗}30mm, 50µm)) and the isomers numbered according to the order of elution:
**Stereoisomer 2:** (+)-2-(4-Methylbenzyl)-7-(tetrahydro-2*H*-pyran-4-yl)-3-((tetrahydrofuran-3-yl)methyl)imidazo[5,1-*f*][1,2,4]triazin-4(3*H*)-one
   10 mg (15%).
   ¹H NMR (CD₃OD, 400 MHz): *δ* 7.73 (s, 1H), 7.24-7.19 (m, 4H), 4.19 (s, 2H), 4.06-3.86 (m, 5H), 3.75-3.67 (m, 2H), 3.60-3.45 (m, 4H), 2.61-2.52 (m, 1H), 2.36 (m, 3H), 2.05-1.90 (m, 5H), 1.72-1.63 (m, 1H).
   LC-MS: (*m*/*z)* 409.2 (MH⁺) t_{R} (minutes, method 4) = 2.28 minutes
   [α]_{D}²⁰ +64 (c = 0.10, MeOH)
**Stereoisomer 1:** (-)-2-(4-Methylbenzyl)-7-(tetrahydro-2*H*-pyran-4-yl)-3-((tetrahydrofuran-3-yl)methyl)imidazo[5,1-*f*][1,2,4]triazin-4(3*H*)-one
   10 mg (15%)
   ¹H NMR (CD₃OD, 400 MHz): *δ* 7.71 (s, 1H), 7.21-7.19 (m, 4H), 4.17 (s, 2H), 4.04-4.00 (m, 2H), 3.99-3.84 (m, 3H), 3.74-3.65 (m, 2H), 3.59-3.44 (m, 4H), 2.60-2.48 (m, 1H), 2.32 (m, 3H), 2.03-1.88 (m, 5H), 1.70-1.61 (m, 1H).
   LC-MS: (*m*/*z)* 409.2 (MH⁺) t_{R} (minutes, method 4) = 2.28 minutes
   [α]_{D}²¹-71 (c = 0.10, MeOH)

### Example 13

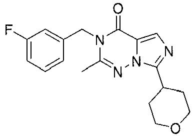

### 3-(3-Fluorobenzyl)-2-methyl-7-(tetrahydro-2H-pyran-4-yl)imidazo[5,1-f][1,2,4]triazin-4(3H)-one:

To a solution 2-methyl-7-(tetrahydro-2*H*-pyran-4-yl)imidazo[5,1-*f*][1,2,4]triazin-4(3*H*)-one (60 mg, 0.26 mmol) in dry DMF (2 mL) was added 1-(bromomethyl)-3-fluorobenzene (73 mg, 0.38 mmol) and K₂CO₃ (71 mg, 0.51 mmol). The mixture was heated at 60°C for 16 hrs. Then it was concentrated *in vacuo* and purified by preparative HPLC to give 3-(3-fluorobenzyl)-2-methyl-7-(tetrahydro-2*H*-pyran-4-yl)imidazo[5,1-*f*][1,2,4]triazin-4(3*H*)-one 27 mg (31%).
¹H NMR (CDCl₃, 400MHz): *δ* 7.86 (s, 1H), 7.34-7.30 (m, 1H), 7.01-6.99 (m, 2H), 6.93-6.91 (m, 1H), 5.22 (s, 2H), 4.11-4.08 (m, 2H), 3.62-3.56 (m, 2H), 3.47-3.44 (m, 1H), 2.39 (s, 3H), 2.17-2.07 (m, 2H), 1.94-1.91 (m, 2H).
LC-MS: (*m*/*z)* 343.1 (MH⁺) t_{R} (minutes, method 3) = 2.56 minutes

### Example 14:

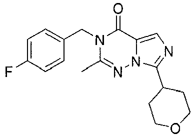

### 3-(4-Fluorobenzyl)-2-methyl-7-(tetrahydro-2H-pyran-4-yl)imidazo[5,1-f][1,2,4]triazin-4(3H)-one:

To a solution of 2-methyl-7-(tetrahydro-2*H*-pyran-4-yl)imidazo[5,1-*f*][1,2,4]triazin-4(3*H*)-one (40 mg, 0.17 mmol) in dry DMF (2 mL) was added 1-(bromomethyl)-4-fluorobenzene (48 mg, 0.26 mmol) and K₂CO₃ (47 mg, 0.34 mmol). The mixture was heated at 60°C for 16 hrs and then concentrated *in vacuo* and purified by flash chromatography using a gradient of dichloromethane and methanol to give 3-(4-fluorobenzyl)-2-methyl-7-(tetrahydro-2*H*-pyran-4-yl)imidazo[5,1-*f*][1,2,4]triazin-4(3*H*)-one 28 mg (48%).
¹H NMR (CDCl₃, 400 MHz): *δ* 7.85 (s, 1H), 7.25-7.21 (m, 2H), 7.06-7.02 (m, 2H), 5.19 (s, 2H), 4.10-4.07 (m, 2H), 3.61-3.55 (m, 2H), 3.45-3.40 (m, 1H), 2.39 (s, 3H), 2.16-2.05 (m, 2H), 1.92-1.89 (m, 2H).
LC-MS: (*m*/*z)* 343.1 (MH⁺) t_{R} (minutes, method 3) = 2.55 minutes

### Example 15:

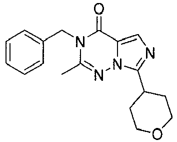

### 3-Benzyl-2-methyl-7-(tetrahydro-2H-pyran-4-yl)imidazo[5,1-f][1,2,4]triazin-4(3H)-one:

To a solution of 2-methyl-7-(tetrahydro-2*H*-pyran-4-yl)imidazo[5,1-*f*][1,2,4]triazin-4(3*H*)-one (60 mg, 0.26 mmol) in dry DMF (2 mL) was added (bromomethyl)benzene (66 mg, 0.38 mmol) and K₂CO₃ (71 mg, 0.51 mmol). The mixture was heated at 60°C for 16 hrs and then concentrated *in vacuo* and purified by preparative HPLC to give 3-benzyl-2-methyl-7-(tetrahydro-2*H*-pyran-4-yl)imidazo[5,1-*f*][1,2,4]triazin-4(3*H*)-one 35mg (42%).
¹H NMR (CDCl₃, 400MHz): *δ* 7.86 (s, 1H), 7.38-7.31 (m, 3H), 7.23-7.21 (m, 2H), 5.24 (s, 2H), 4.11-4.09 (m, 2H), 3.62-3.57 (m, 2H), 3.46-3.41 (m, 1H), 2.39 (s, 3H), 2.16-2.05 (m, 2H), 1.94-1.91 (m, 2H).
LC-MS: (*m*/*z*) 325.2 (MH⁺) t_{R} (minutes, method 4) = 2.08 minutes

### Example 16:

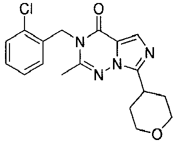

### 3-(2-Chlorobenzyl)-2-methyl-7-(tetrahydro-2H-pyran-4-yl)imidazo[5,1-f][1,2,4]triazin-4(3H)-one:

To a solution of 2-methyl-7-(tetrahydro-2*H*-pyran-4-yl)imidazo[5,1-*f*][1,2,4]triazin-4(3*H*)-one (60 mg, 0.26 mmol) in dry DMF (2 mL) was added 1-(bromomethyl)-2-chlorobenzene (79 mg, 0.38 mmol) and K₂CO₃ (71 mg, 0.51 mmol). The mixture was heated at 60°C for 16 hrs and then concentrated *in vacuo* and purified by preparative HPLC to give 3-(2-chlorobenzyl)-2-methyl-7-(tetrahydro-2*H*-pyran-4-yl)imidazo[5,1-*f*][1,2,4]triazin-4(3*H*)-one 45 mg (49%).
¹H NMR (CDCl₃, 400 MHz): *δ* 7.88 (s, 1H), 7.45-7.43 (m, 1H), 7.26-7.22 (m, 2H), 6.96-6.94 (m, 1H), 5.34 (s, 2H), 4.12-4.10 (m, 2H), 3.63-3.58 (m, 2H), 3.48-3.43 (m, 1H), 2.34 (s, 3H), 2.17-2.08 (m, 2H), 1.96-1.93 (m, 2H).
LC-MS: (*m*/*z)* 359.1 (MH⁺) t_{R} (minutes, method 3) = 2.70 minutes

### Example 17:

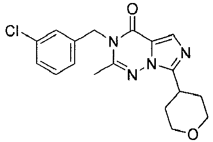

### 3-(3-Chlorobenzyl)-2-methyl-7-(tetrahydro-2H-pyran-4-yl)imidazo[5,1-f][1,2,4]triazin-4(3H)-one:

To a solution of 2-methyl-7-(tetrahydro-2*H*-pyran-4-yl)imidazo[5,1-*f*][1,2,4]triazin-4(3*H*)-one (60 mg, 0.26 mmol) in dry DMF (2 mL) was added 1-(bromomethyl)-3-chlorobenzene (79 mg, 0.38 mmol) and K₂CO₃ (71 mg, 0.51 mmol). The mixture was heated at 60°C for 16 hrs and then concentrated *in vacuo.* The crude product was purified by preparative HPLC to give 3-(3-chlorobenzyl)-2-methyl-7-(tetrahydro-2*H*-pyran-4-yl)imidazo[5,1-*f*][1,2,4]triazin-4(3*H*)-one 36 mg (39%).
**¹H NMR** (CDCl₃, 400 MHz): δ 7.86 (s, 1H), 7.32-7.28 (m, 2H), 7.20 (m, 1H), 7.11-7.10 (m, 1H), 5.20 (s, 2H), 4.11-4.08 (m, 2H), 3.62-3.56 (m, 2H), 3.47-3.43 (m, 1H), 2.39 (s, 3H), 2.15-2.06 (m, 2H), 1.94-1.91 (m, 2H).
LC-MS: (m/z) 359.1 (MH⁺) t_{R} (minutes, method 3) = 2.70 minutes

### Example 18:

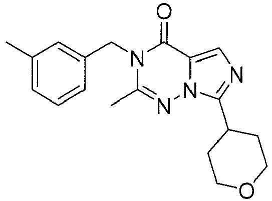

### 2-Methyl-3-(3-methylbenzyl)-7-(tetrahydro-2H-pyran-4-yl)imidazo[5,1-f][1,2,4]triazin-4(3H)-one:

To a solution of 2-methyl-7-(tetrahydro-2*H*-pyran-4-yl)imidazo[5,1-*f*][1,2,4]triazin-4(3*H*)-one (60 mg, 0.26 mmol) in dry DMF (2 mL) was added 1-(bromomethyl)-3-methylbenzene (71 mg, 0.38 mmol) and K₂CO₃ (71 mg, 0.51 mmol). The mixture was heated at 60°C for 16 hrs and then concentrated *in vacuo.* The crude product was purified by preparative HPLC to give 2-methyl-3-(3-methylbenzyl)-7-(tetrahydro-2*H*-pyran-4-yl)imidazo[5,1-*f*][1,2,4]triazin-4(3*H*)-one 45 mg (52%).
¹H NMR (CDCl₃, 400MHz): δ 7.86 (s, 1H), 7.25-7.22 (m, 1H), 7.11 (d, *J* = 7.2 Hz, 1H), 7.01-6.99 (m, 2H), 5.20 (s, 2H), 4.11-4.08 (m, 2H), 3.63-3.56 (m, 2H), 3.47-3.44 (m, 1H), 2.40 (s, 3H), 2.33 (s, 3H), 2.13-2.06 (m, 2H), 1.95-1.91 (m, 2H).
LC-MS: *(m*/*z)* 339.2 (MH⁺) t_{R} (minutes, method 4) = 2.24 minutes

### Example 19

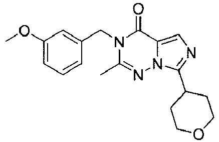

### 3-(3-Methoxybenzyl)-2-methyl-7-(tetrahydro-2H-pyran-4-yl)imidazo[5,1-f][1,2,4]triazin-4(3H)-one:

To a solution of 2-methyl-7-(tetrahydro-2*H*-pyran-4-yl)imidazo[5,1-*f*][1,2,4]triazin-4(3*H*)-one (60 mg, 0.26 mmol) in dry DMF (2 mL) was added 1-(bromomethyl)-3-methoxybenzene (77 mg, 0.38 mmol) and K₂CO₃ (71 mg, 0.51 mmol). The mixture was heated at 60°C for 16 hrs and then concentrated *in vacuo.* The crude product was concentrated and purified by preparative HPLC to give 3-(3-methoxybenzyl)-2-methyl-7-(tetrahydro-2H-pyran-4-yl)imidazo[5,1-*f*][1,2,4]triazin-4(3*H*)-one 40 mg (44%).
¹H NMR (CDCl₃, 400 MHz): *δ* 7.85 (s, 1H), 7.29-7.25 (m, 1H), 6.83 (d, *J* = 8.0 Hz, 1H), 6.78 (d, *J* = 8.0 Hz, 1H), 6.74 (s, 1H), 5.21 (s, 2H), 4.11-4.09 (m, 2H), 3.79 (s, 3H), 3.62-3.57 (m, 2H), 3.45-3.41 (m, 1H), 2.39 (s, 3H), 2.15-2.06 (m, 2H), 1.94-1.91 (m, 2H).
LC-MS: *(m*/*z)* 355.2 (MH⁺) t_{R} (minutes, method 4) = 2.11 minutes

### Example 20

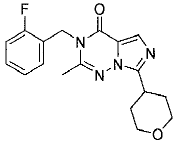

### 3-(2-Fluorobenzyl)-2-methyl-7-(tetrahydro-2H-pyran-4-yl)imidazo[5,1-f][1,2,4]triazin-4(3H)-one:

To a solution of 2-methyl-7-(tetrahydro-2*H*-pyran-4-yl)imidazo[5,1-*f*][1,2,4]triazin-4(3*H*)-one (60 mg, 0.26 mmol) in dry DMF (2 mL) was added 1-(bromomethyl)-2-fluorobenzene (73 mg, 0.38 mmol) and K₂CO₃ (71 mg, 0.51 mmol). The mixture was heated at 60°C for 16 hrs and then concentrated *in vacuo.* The crude product was purified by preparative HPLC to give 3-(2-fluorobenzyl)-2-methyl-7-(tetrahydro-2*H*-pyran-4-yl)imidazo[5,1-*f*][1,2,4]triazin-4(3*H*)-one 35 mg (40%).
¹H NMR (CDCl₃, 400 MHz): *δ* 7.84 (s, 1H), 7.31-7.28 (m, 1H), 7.14-7.07 (m, 3H), 5.27 (s, 2H), 4.10-4.07 (m, 2H), 3.61-3.55 (m, 2H), 3.46-3.42 (m, 1H), 2.39 (s, 3H), 2.13-2.04 (m, 2H), 1.93-1.89 (m, 2H).
LC-MS: (m/z) 343.1 (MH⁺) t_{R} (minutes, method 3) = 2.53 minutes

### Example 21

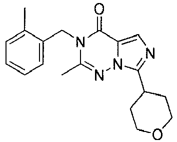

### 2-Methyl-3-(2-methylbenzyl)-7-(tetrahydro-2H-pyran-4-yl)imidazo[5,1-f][1,2,4]triazin-4(3H)-one:

To a solution of 2-methyl-7-(tetrahydro-2*H*-pyran-4-yl)imidazo[5,1-*f*][1,2,4]triazin-4(3*H*)-one (60 mg, 0.26 mmol) in dry DMF (2 mL) was added 1-(bromomethyl)-2-methylbenzene (71 mg, 0.38 mmol) and K₂CO₃ (71 mg, 0.51 mmol). The mixture was heated at 60°C for 16 hrs and then concentrated *in vacuo.* The crude product was purified by preparative HPLC to give 2-methyl-3-(2-methylbenzyl)-7-(tetrahydro-2*H*-pyran-4-yl)imidazo[5,1-*f*][1,2,4]triazin-4(3*H*)-one 33 mg (38%).
¹H NMR (CDCl₃, 400 MHz): *δ* 7.86 (s, 1H), 7.23-7.15 (m, 3H), 6.78 (d, *J* = 7.6 Hz, 1H), 5.19 (s, 2H), 4.13-4.10 (m, 2H), 3.64-3.58 (m, 2H), 3.48-3.46 (m, 1H), 2.40 (s, 3H), 2.33 (s, 3H), 2.19-2.10 (m, 2H), 1.97-1.94 (m, 2H).
LC-MS: (m/z) 339.1 (MH⁺) t_{R} (minutes, method 3) = 2.61 minutes

### Example 22

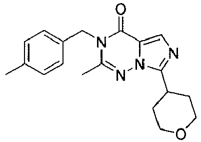

### 2-Methyl-3-(4-methylbenzyl)-7-(tetrahydro-2H-pyran-4-yl)imidazo[5,1-f][1,2,4]triazin-4(3H)-one:

To a solution of 2-methyl-7-(tetrahydro-2*H*-pyran-4-yl)imidazo[5,1-*f*][1,2,4]triazin-4(3*H*)-one (60 mg, 0.26 mmol) in dry DMF (2 mL) was added 1-(bromomethyl)-4-methylbenzene (71 mg, 0.38 mmol) and K₂CO₃ (71 mg, 0.51 mmol). The mixture was heated at 60°C for 16 hrs and then concentrated *in vacuo.* The crude product was purified by preparative HPLC to give 2-methyl-3-(4-methylbenzyl)-7-(tetrahydro-2*H*-pyran-4-yl)imidazo[5,1-*f*][1,2,4]triazin-4(3*H*)-one 35 mg, (40%).
¹H NMR (CDCl₃, 400 MHz): *δ* 7.83 (s, 1H), 7.15-7.08 (m, 4H), 5.18 (s, 2H), 4.09-4.07 (m, 2H), 3.60-3.54 (m, 2H), 3.44-3.39 (m, 1H), 2.38 (s, 3H), 2.32 (s, 3H), 2.14-2.05 (m, 2H), 1.92-1.89 (m, 2H).
LC-MS: (m/z) 339.2 (MH⁺) t_{R} (minutes, method 3) = 2.65 minutes

### Example 23

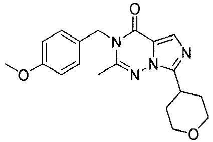

### 3-(4-Methoxybenzyl)-2-methyl-7-(tetrahydro-2H-pyran-4-yl)imidazo[5,1-f][1,2,4]triazin-4(3H)-one:

To a solution of 2-methyl-7-(tetrahydro-2*H*-pyran-4-yl)imidazo[5,1-*f*][1,2,4]triazin-4(3*H*)-one (60 mg, 0.26 mmol) in dry DMF (2 mL) was added 1-(bromomethyl)-4-methoxybenzene (77 mg, 0.38 mmol) and K₂CO₃ (71 mg, 0.51 mmol). The mixture was heated at 60°C for 16 hrs and then concentrated *in vacuo.* The crude product was purified by preparative HPLC to give 3-(4-methoxybenzyl)-2-methyl-7-(tetrahydro-2*H*-pyran-4-yl)imidazo[5,1-*f*][1,2,4]triazin-4(3*H*)-one 35 mg (38%).
¹H NMR (CDCl₃, 400 MHz): δ 7.85 (s, 1H), 7.17 (d, *J* = 8.8 Hz, 2H), 6.89-6.86 (m, 2H), 5.17 (s, 2H), 4.11-4.08 (m, 2H), 3.79 (s, 3H), 3.62-3.56 (m, 2H), 3.45-3.41 (m, 1H), 2.41 (s, 3H), 2.16-2.08 (m, 2H), 1.93-1.89 (m, 2H).
LC-MS: (*m*/*z*) 355.1 (MH⁺) t_{R} (minutes, method 3) = 2.49 minutes

### Example 24

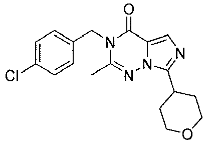

### 3-(4-Chlorobenzyl)-2-methyl-7-(tetrahydro-2H-pyran-4-yl)imidazo[5,1-f][1,2,4]triazin-4(3H)-one:

To a solution of 2-methyl-7-(tetrahydro-2*H*-pyran-4-yl)imidazo[5,1-*f*][1,2,4]triazin-4(3*H*)-one (60 mg, 0.26 mmol) in dry DMF (2 mL) was added 1-(bromomethyl)-4-chlorobenzene (79 mg, 0.38 mmol) and K₂CO₃ (71 mg, 0.51 mmol). The mixture was heated at 60°C for 16 hrs. The mixture was concentrated and purified by preparative HPLC to give 3-(4-chlorobenzyl)-2-methyl-7-(tetrahydro-2*H*-pyran-4-yl)imidazo[5,1-*f*][1,2,4]triazin-4(3*H*)-one 35 mg (38%).
¹H NMR (CDCl₃, 400 MHz): *δ* 7.84 (s, 1H), 7.32 (d, *J* = 8.4 Hz, 2H), 7.15 (d, *J* = 8.4 Hz, 2H), 5.18 (s, 2H), 4.10-4.07 (m, 2H), 3.60-3.55 (m, 2H), 3.46-3.41 (m, 1H), 2.37 (s, 3H), 2.13-2.04 (m, 2H), 1.92-1.89 (m, 2H).
LC-MS: *(m*/*z)* 359.1 (MH⁺) t_{R} (minutes, method 3) = 2.70 minutes

### Example 25

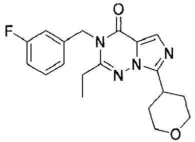

### 2-Ethyl-3-(3-fluorobenzyl)-7-(tetrahydro-2H-pyran-4-yl)imidazo[5,1-f][1,2,4]triazin-4(3H)-one:

A mixture of 2-ethyl-7-(tetrahydro-2*H*-pyran-4-yl)imidazo[5,1-*f*][1,2,4]triazin-4(3*H*)-one (70 mg, 0.28 mmol, 1.0 eq), 1-(bromomethyl)-3-fluoro-benzene (80 mg, 0.42 mmol) and K₂CO₃ (78 mg, 0.56 mmol) in dry DMF (5 mL) was heated 60°C for 2 hrs. The reaction was cooled and poured into H₂O (5 mL) and extracted with ethyl acetate (3×10 mL). The combined organic phase was washed with brine (50 mL), dried over Na₂SO₄ and concentrated *in vacuo.* The residue was purified by preparative HPLC to give 2-ethyl-3-(3-fluorobenzyl)-7-(tetrahydro-2*H*-pyran-4-yl)imidazo[5,1-*f*][1,2,4]triazin-4(3*H*)-one 52 mg, (51%).
¹H NMR (CDCl₃, 400 MHz): δ 7.86 (s, 1H), 7.35-7.30 (m, 1H), 7.02-6.97 (m, 2H), 6.90 (d, *J* = 9.6 Hz, 1H), 5.24 (s, 2H), 4.10 (d, *J* = 10.4 Hz, 2H), 3.63-3.60 (m, 2H), 3.57-3.46 (m, 1H), 2.69-2.64 (q, *J* = 7.2 Hz,2H), 2.55-2.51 (m, 2H), 1.98-1.75 (m, 2H), 1.31-1.27 (t, *J* = 7.2 Hz,3H).
LC-MS: (*m*/*z*) 357.2 (MH⁺) t_{R} (minutes, method 3) = 2.714 minutes

### Example 26

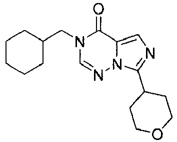

### 3-(Cyclohexylmethyl)-7-(tetrahydro-2H-pyran-4-yl)imidazo[5,1-f][1,2,4]triazin-4(3H)-one:

To a solution of 7-(tetrahydro-2*H*-pyran-4-yl)imidazo[5,1-*f*][1,2,4]triazin-4(3*H*)-one (150 mg, 0.68 mmol) in anhydrous dimethylformamide (5 mL) was added K₂CO₃ (141 mg, 1.0 mmol) and bromomethylcyclohexane (145 mg, 0.82 mmol). The mixture was stirred at 65°C for 16 h. The reaction was cooled and diluted with water (10 mL). The mixture was extracted with ethyl acetate (2x20 mL). The combined organic phases were washed with brine, dried over Na₂SO₄ and concentrated in vacuo. The residue was purified by preparative TLC, using dichloromethane/methanol=20/1 as eluent, to afford 3-(cyclohexylmethyl)-7-(tetrahydro-2*H-*pyran-4-yl)imidazo[5,1-*f*][1,2,4]triazin-4(3*H*)-one 15 mg (7%).
¹H NMR (CD₃OD, 400 MHz): δ 7.96 (s, 1H), 7.76 (s, 1H), 4.09-4.05 (m, 2H), 3.78 (d, *J* = 6.8 Hz, 2H), 3.64-3.33 (m, 3H), 2.05-1.91 (m, 4H), 1.79-1.70 (m, 6H), 1.31-1.25 (m, 3H), 1.08-1.05 (m, 2H).
LC-MS: (m/z) 317.2 (MH⁺) t_{R} (minutes, method 4) = 2.26 minutes

### Example 27:

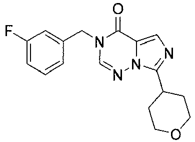

### 3-(3-Fluorobenzyl)-7-(tetrahydro-2H-pyran-4-yl)imidazo[5,1-f][1,2,4]triazin-4(3H)-one:

To a solution of 7-(tetrahydro-2*H*-pyran-4-yl)imidazo[5,1-*f*][1,2,4]triazin-4(3*H*)-one (150 mg, 0.68 mmol) in anhydrous dimethylformamide (5 mL) was added K₂CO₃ (94 mg, 0.68 mmol) and 1-(bromomethyl)-3-fluorobenzene (154 mg, 0.82 mmol). The reaction mixture was stirred at 65°C for 16 hrs. The mixture was cooled to room temperature and diluted with water (10 mL). The crude mixture was extracted with ethyl acetate (2×20 mL). The combined organic phases were washed with brine, dried over Na₂SO₄ and concentrated *in vacuo.* The crude product was purified by preparative TLC, using dichloromethane/methanol 20:1 as eluent, to afford 3-(3-fluorobenzyl)-7-(tetrahydro-2*H*-pyran-4-yl)imidazo[5,1-*f*][1,2,4]triazin-4(3H)-one 15 mg (7%).
¹H NMR (CD₃OD, 400 MHz): δ 8.11 (s, 1H), 7.79 (s, 1H), 7.43-7.38 (m, 1H), 7.24-7.17 (m, 2H), 7.10-7.08 (m, 1H), 5.14 (s, 2H), 4.08-4.04 (m, 2H), 3.64-3.55 (m, 3H), 2.05-1.91 (m, 4H).
LC-MS: *(m*/*z)* 329.1 (MH⁺) t_{R} (minutes, method 4) = 2.04 minutes

### Example 28:

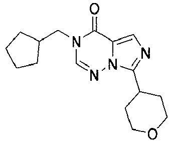

### 3-(Cyclopentylmethyl)-7-(tetrahydro-2H-pyran-4-yl)imidazo[5,1-f][1,2,4]triazin-4(3H)-one:

A mixture of 7-(tetrahydro-2*H*-pyran-4-yl)imidazo[5,1-*f*][1,2,4]triazin-4(3*H*)-one (200 mg, 0.908 mmol), (bromomethyl)cyclopentane (178 mg, 1.1 mmol) and K₂CO₃ (188 mg, 1.4 mmol) in DMF (5 mL) was stirred at 70°C for 16 hrs. The reaction mixture was filtered. The residue was purified with preparative HPLC to give 3-(cyclopentylmethyl)-7-(tetrahydro-2H-pyran-4-yl)imidazo[5,1-*f*][1,2,4]triazin-4(3*H*)-one 35 mg (13%).
¹H NMR (CD₃OD, 400 MHz): δ8.02 (s, 1H), 7.76 (s, 1H), 4.08-4.05 (m, 2H), 3.89 (d, *J* = 7.6 Hz, 2H), 3.65-3.55 (m, 3H), 2.41-2.33 (m, 1H), 2.09-1.91 (m, 4H), 1.82-1.70 (m, 6H), 1.34-1.33 (m, 2H).
LC-MS: (m/z) 303.1 (MH⁺) t_{R} (minutes, method 4) = 2.39 minutes

### Example 29

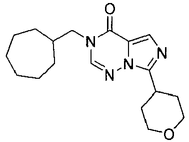

### 3-(Cycloheptylmethyl)-7-(tetrahydro-2H-pyran-4-yl)imidazo[5,1-f][1,2,4]triazin-4(3H)-one:

To a solution of 7-(tetrahydro-2*H*-pyran-4-yl)imidazo[5,1-*f*][1,2,4]triazin-4(3*H*)-one (300 mg, 1.36 mmol), cycloheptylmethyl methanesulfonate (450 mg, 2.2 mmol) in anhydrous dimethylformamide (5 mL) was added K₂CO₃ (376 mg, 2.72 mmol). The reaction was stirred at 100°C for 16 hrs. The reaction mixture was cooled and filtered. The filtrate was purified by preparative HPLC to afford 3-(cycloheptylmethyl)-7-(tetrahydro-2*H*-pyran-4-yl)imidazo[5,1-*f*][1,2,4]triazin-4(3*H*)-one 50 mg (11%).
¹H NMR (CD₃OD, 400 MHz): δ 7.99 (s, 1H), 7.76 (s, 1H), 4.09-4.05 (m, 2H), 3.78 (d, J = 8.0 Hz, 2H), 3.65-3.37 (m, 3H), 2.06-1.95 (m, 5H), 1.74-1.52 (m, 10H), 1.30-1.27 (m, 2H).
LC-MS: (m/z) 331.2 (MH⁺) t_{R} (minutes, method 3) = 2.80 minutes

### IN VITRO TESTING

### PDE1 INHIBITION ASSAY

PDE1A, PDE1B and PDE1C assays were performed as follows: the assays was performed in 60 µL samples containing a fixed amount of the PDE1 enzym1 (sufficient to convert 20-25% of the cyclic nucleotide substrate), a buffer (50 mM HEPES pH 7.6; 10 mM MgCl₂; 0.02% Tween20), 0.1 mg/mL BSA, 15 nM tritium labelled cAMP and varying amounts of inhibitors. Reactions were initiated by addition of the cyclic nucleotide substrate, and reactions were allowed to proceed for 1 hr at room temperature before being terminated through mixing with 20 µL (0.2 mg) yttrium silicate SPA beads (PerkinElmer). The beads were allowed to settle for 1 hr in the dark before the plates were counted in a Wallac 1450 Microbeta counter. The measured signals were converted to activity relative to an uninhibited control (100%) and IC₅₀ values were calculated using XIFit (model 205, IDBS).

### PDE9 INHIBITION ASSAY

A PDE9 assay may for example, be performed as follows: The assay is performed in 60 µL samples containing a fixed amount of the relevant PDE enzyme (sufficient to convert 20-25% of the cyclic nucleotide substrate), a buffer (50 mM HEPES7.6; 10mM MgCl₂; 0.02% Tween20), 0.1 mg/mL BSA, 225 pCi of ³H-labelled cyclic nucleotide substrate, tritium labeled cAMP to a final concentration of 5 nM and varying amounts of inhibitors. Reactions are initiated by addition of the cyclic nucleotide substrate, and reactions are allowed to proceed for one hr at room temperature before being terminated through mixing with 15 µL 8 mg/mL yttrium silicate SPA beads (Amersham). The beads are allowed to settle for one hr in the dark before the plates are counted in a Wallac 1450 Microbeta counter. The measured signal can be converted to activity relative to an uninhibited control (100 %) and IC₅₀ values can be calculated using the Xlfit extension to EXCEL.

In the context of the present invention the assay was performed in 60 uL assay buffer (50 mM HEPES pH 7.6; 10mM MgCl₂; 0.02% Tween20) containing enough PDE9 to convert 20-25% of 10 nM ³H-cAMP and varying amounts of inhibitors. Following a 1 hr incubation the reactions were terminated by addition of 15 uL 8 mg/mL yttrium silicate SPA beads (Amersham). The beads were allowed to settle for one hr in the dark before the plates were counted in a Wallac 1450 Microbeta counter.

## Claims

1. A compound according to formula (I) 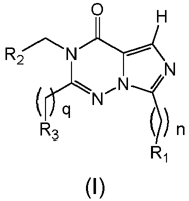 wherein
n is 0 or 1;
q is 0 or 1;
R1 is selected from the group consisting of linear or branched C₁-C₈ alkyl, saturated monocyclic C₃-C₈ cycloalkyl, tetrahydrofuranyl and tetrahydropyranyl; all of which can be substituted one or more times with fluorine;
R2 is selected from the group consisting of hydrogen, linear or branched C₁-C₈ alkyl, saturated monocyclic C₃-C₈ cycloalkyl, tetrahydrofuranyl and tetrahydropyranyl; or
R2 is phenyl or 5 or 6-membered heteroaryl, all of which can be substituted with one or more substituents selected from the group consisting of halogen, C₁-C₃ alkyl and methoxy; or
R2 is a saturated monocyclic C₃-C₈ cycloalkyl, substituted one or two times with methyl;
R3 is selected from the group consisting of hydrogen, C₁-C₅ alkyl, and phenyl; or
R3 is selected from the group consisting of C₁-C₅ alkyl substituted one or more times with fluorine; or
R3 is phenyl substituted one or more times with C₁-C₃ alkyl;
with the proviso that R2 and R3 cannot be hydrogen in the same molecule;
and tautomers and pharmaceutically acceptable addition salts thereof.

2. The compound according to claim 1, wherein
n is 0 and q is 0;
R1 is selected from tetrahydrofuranyl and tetrahydropyranyl;
R2 is selected from the group consisting of linear or branched C₁-C₈ alkyl, phenyl, saturated monocyclic C₃-C₈ cycloalkyl, tetrahydrofuranyl and tetrahydropyranyl; or
R2 is selected from the group consisting of phenyl substituted with a substituent selected from the group consisting of halogen, C₁-C₃ alkyl and methoxy; and saturated monocyclic C₃-C₈ cycloalkyl, substituted one or two times with methyl;
R3 is selected from the group consisting of hydrogen and C₁-C₅ alkyl; or
R3 is selected from methyl substituted one, two or three times with fluorine; and ethyl substituted one, two or three times with fluorine.

3. The compound of claim 1, wherein n is 0 and R1 is tetrahydropyranyl.

4. The compound of claim 1, wherein R2 is phenyl.

5. The compound of claim 1, wherein R2 is phenyl substituted with a substituent selected from the group consisting of fluorine, chlorine and methyl.

6. The compound of claim 1, wherein R3 is methyl or ethyl, optionally substituted one, two or three times with fluorine.

7. The compound of claim 1, wherein R3 is hydrogen, methyl or ethyl; and
R2 is selected from the group consisting of linear or branched C₁-C₈ alkyl, saturated monocyclic C₃-C₈ cycloalkyl, tetrahydrofuranyl and tetrahydropyranyl; or
R2 is phenyl or 5 or 6-membered heteroaryl, all of which can be substituted with one or more substituents selected from the group consisting of halogen, C₁-C₃ alkyl and methoxy; or
R2 is a saturated monocyclic C₃-C₈ cycloalkyl, substituted one or two times with methyl.

8. The compound of claim 1, wherein the compound is selected from the group consisting of:
3-(cyclohexylmethyl)-2-methyl-7-(tetrahydro-2H-pyran-4-yl)imidazo[5,1-f][1,2,4]triazin-4(3H)-one;
3-methyl-2-(4-methylbenzyl)-7-(tetrahydro-2H-pyran-4-yl)imidazo[5,1-f][1,2,4]triazin-4(3H)-one;
3-ethyl-2-(4-methylbenzyl)-7-(tetrahydro-2H-pyran-4-yl)imidazo[5,1-f][1,2,4]triazin-4(3H)-one;
2-(4-methylbenzyl)-3-propyl-7-(tetrahydro-2H-pyran-4-yl)imidazo[5,1-f][1,2,4]triazin-4(3H)-one;
3-isobutyl-2-(4-methylbenzyl)-7-(tetrahydro-2H-pyran-4-yl)imidazo[5,1-f][1,2,4]triazin-4(3H)-one;
3-(cyclopentylmethyl)-2-(4-methylbenzyl)-7-(tetrahydro-2H-pyran-4-yl)imidazo[5,1-f][1,2,4]triazin-4(3H)-one;
3-(cyclohexylmethyl)-2-(4-methylbenzyl)-7-(tetrahydro-2H-pyran-4-yl)imidazo[5,1-f][1,2,4]triazin-4(3H)-one;
3-(cyclopropylmethyl)-2-(4-methylbenzyl)-7-(tetrahydro-2H-pyran-4-yl)imidazo[5,1-f][1,2,4]triazin-4(3H)-one
2-(4-methylbenzyl)-7-(tetrahydro-2H-pyran-4-yl)-3-((tetrahydro-2H-pyran-4-yl)methyl)imidazo[5,1-f][1,2,4]triazin-4(3H)-one;
3-(cyclobutylmethyl)-2-(4-methylbenzyl)-7-(tetrahydro-2H-pyran-4-yl)imidazo[5,1-f][1,2,4]triazin-4(3H)-one;
cis-2-(4-methylbenzyl)-3-((4-methylcyclohexyl)methyl)-7-(tetrahydro-2H-pyran-4-yl)imidazo[5,1-f][1,2,4]triazin-4(3H)-one;
trans-2-(4-methylbenzyl)-3-((4-methylcyclohexyl)methyl)-7-(tetrahydro-2H-pyran-4-yl)imidazo[5,1-f][1,2,4]triazin-4(3H)-one;
(-)-2-(4-methylbenzyl)-7-(tetrahydro-2H-pyran-4-yl)-3-((tetrahydrofuran-3-yl)methyl)imidazo[5,1-f][1,2,4]triazin-4(3H)-one;
(+)-2-(4-methylbenzyl)-7-(tetrahydro-2H-pyran-4-yl)-3-((tetrahydrofuran-3-yl)methyl)imidazo[5,1-f][1,2,4]triazin-4(3H)-one;
3-(3-fluorobenzyl)-2-methyl-7-(tetrahydro-2H-pyran-4-yl)imidazo[5,1-f][1,2,4]triazin-4(3H)-one;
3-(4-fluorobenzyl)-2-methyl-7-(tetrahydro-2H-pyran-4-yl)imidazo[5,1-f][1,2,4]triazin-4(3H)-one;
3-benzyl-2-methyl-7-(tetrahydro-2H-pyran-4-yl)imidazo[5,1-f][1,2,4]triazin-4(3H)-one;
3-(2-chlorobenzyl)-2-methyl-7-(tetrahydro-2H-pyran-4-yl)imidazo[5,1-f][1,2,4]triazin-4(3H)-one;
3-(3-chlorobenzyl)-2-methyl-7-(tetrahydro-2H-pyran-4-yl)imidazo[5,1-f][1,2,4]triazin-4(3H)-one;
2-methyl-3-(3-methylbenzyl)-7-(tetrahydro-2H-pyran-4-yl)imidazo[5,1-f][1,2,4]triazin-4(3H)-one;
3-(3-methoxybenzyl)-2-methyl-7-(tetrahydro-2H-pyran-4-yl)imidazo[5,1-f][1,2,4]triazin-4(3H)-one;
3-(2-fluorobenzyl)-2-methyl-7-(tetrahydro-2H-pyran-4-yl)imidazo[5,1-f][1,2,4]triazin-4(3H)-one;
2-methyl-3-(2-methylbenzyl)-7-(tetrahydro-2H-pyran-4-yl)imidazo[5,1-f][1,2,4]triazin-4(3H)-one;
2-methyl-3-(4-methylbenzyl)-7-(tetrahydro-2H-pyran-4-yl)imidazo[5,1-f][1,2,4]triazin-4(3H)-one;
3-(4-methoxybenzyl)-2-methyl-7-(tetrahydro-2H-pyran-4-yl)imidazo[5,1-f][1,2,4]triazin-4(3H)-one;
3-(4-chlorobenzyl)-2-methyl-7-(tetrahydro-2H-pyran-4-yl)imidazo[5,1-f][1,2,4]triazin-4(3H)-one;
2-ethyl-3-(3-fluorobenzyl)-7-(tetrahydro-2H-pyran-4-yl)imidazo[5,1-f][1,2,4]triazin-4(3H)-one;
3-(cyclohexylmethyl)-7-(tetrahydro-2H-pyran-4-yl)imidazo[5,1-f][1,2,4]triazin-4(3H)-one;
3-(3-fluorobenzyl)-7-(tetrahydro-2H-pyran-4-yl)imidazo[5,1-f][1,2,4]triazin-4(3H)-one;
3-(cyclopentylmethyl)-7-(tetrahydro-2H-pyran-4-yl)imidazo[5,1-f][1,2,4]triazin-4(3H)-one; and
3-(cycloheptylmethyl)-7-(tetrahydro-2H-pyran-4-yl)imidazo[5,1-f][1,2,4]triazin-4(3H)-one;
and pharmaceutically acceptable salts of any of these compounds.

9. A compound of any one of claims 1 to 8 for use as a medicament.

10. A pharmaceutical composition comprising a therapeutically effective amount of a compound of any one of claims 1 to 8 and one or more pharmaceutically acceptable carriers, diluents and excipients.

11. A compound of any one of claims 1-8 for use in the treatment of a neurodegenerative disorder, selected from the group consisting of Alzheimer's Disease, Parkinson's Disease and Huntington's Disease or for the treatment of a psychiatric disorder such as Attention Deficit hyperactivity Disorder (ADHD), depression, anxiety, narcolepsy, cognitive impairment and cognitive impairment associated with schizophrenia (CIAS), or another brain disease like restless leg syndrome.

## Patentansprüche

1. Verbindung gemäß Formel (I) 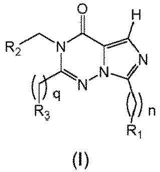 wobei
n 0 oder 1 ist;
q 0 oder 1 ist;
R1 ausgewählt ist aus der Gruppe bestehend aus linearem oder verzweigtem C₁-C₈-Alkyl, gesättigtem monozyklischem C₃-C₈-Cycloalkyl, Tetrahydrofuranyl und Tetrahydropyranyl; von denen alle ein oder mehrere Male mit Fluor substituiert sein können;
R2 ausgewählt ist aus der Gruppe bestehend aus Wasserstoff, linearem oder verzweigtem C₁-C₈-Alkyl, gesättigtem monozyklischem C₃-C₈-Cycloalkyl, Tetrahydrofuranyl und Tetrahydropyranyl; oder
R2 Phenyl oder 5- oder 6-gliedriges Heteroaryl ist, von denen alle mit einem oder mehreren Substituenten substituiert sein können, die ausgewählt sind aus der Gruppe bestehend aus Halogen, C₁-C₃-Alkyl und Methoxy; oder
R2 ein gesättigtes monozyklisches C₃-C₈-Cycloalkyl ist, das ein oder zwei Mal mit Methyl subsituiert ist;
R3 ausgewählt ist aus der Gruppe bestehend aus Wasserstoff, C₁-C₅-Alkyl und Phenyl; oder
R3 ausgewählt ist aus der Gruppe bestehend aus C₁-C₅-Alkyl, das ein oder mehrere Male mit Fluor substituiert ist; oder
R3 Phenyl ist, das ein oder mehrere Male mit C₁-C₃-Alkyl substituiert ist;
mit der Maßgabe, dass R2 und R3 nicht Wasserstoff in dem gleichen Molekül sein können;
und Tautomere und pharmazeutisch annehmbare Additionssalze davon.

2. Verbindung nach Anspruch 1, wobei
n 0 ist und q 0 ist;
R1 ausgewählt ist aus Tetrahydrofuranyl und Tetrahydropyranyl;
R2 ausgewählt ist aus der Gruppe bestehend aus linearem oder verzweigtem C₁-C₈-Alkyl, Phenyl, gesättigtem monozyklischem C₃-C₈-Cycloalkyl, Tetrahydrofuranyl und Tetrahydropyranyl; oder
R2 ausgewählt ist aus der Gruppe bestehend aus Phenyl, das mit einem Substituenten substituiert ist, der ausgewählt ist aus der Gruppe bestehend aus Halogen, C₁-C₃-Alkyl und Methoxy; und gesättigtem monozyklischem C₃-C₈-Cycloalkyl, das ein oder zwei Mal mit Methyl substituiert ist;
R3 ausgewählt ist aus der Gruppe bestehend aus Wasserstoff und C₁-C₅-Alkyl; oder
R3 ausgewählt ist aus Methyl, das ein oder zwei Mal mit Fluor substituiert ist; und Ethyl, das ein, zwei oder drei Mal mit Fluor substituiert ist.

3. Verbindung nach Anspruch 1, wobei n 0 ist und R1 Tetrahydropyranyl ist.

4. Verbindung nach Anspruch 1, wobei R2 Phenyl ist.

5. Verbindung nach Anspruch 1, wobei R2 Phenyl ist, das mit einem Substituenten, ausgewählt aus der Gruppe bestehend aus Fluor, Chlor und Methyl, substituiert ist.

6. Verbindung nach Anspruch 1, wobei R3 Methyl oder Ethyl ist, das optional ein, zwei oder drei Mal mit Fluor substituiert ist.

7. Verbindung nach Anspruch 1, wobei R3 Wasserstoff, Methyl oder Ethyl ist; und
R2 ausgewählt ist aus der Gruppe bestehend aus linearem oder verzweigtem C₁-C₈-Alkyl, gesättigtem monozyklischem C₃-C₈-Cycloalkyl, Tetrahydrofuranyl und Tetrahydropyranyl; oder
R2 Phenyl oder 5- oder 6-gliedriges Heteroaryl ist, von denen alle mit einem oder mehreren Substituenten substituiert sein können, die ausgewählt sind aus der Gruppe bestehend aus Halogen, C₁-C₃-Alkyl und Methoxy; oder
R2 ein gesättigtes monozyklisches C₃-C₈-Cycloalkyl ist, das ein oder zwei Mal mit Methyl subsituiert ist.

8. Verbindung nach Anspruch 1, wobei die Verbindung ausgewählt ist aus der Gruppe bestehend aus:
3-(Cyclohexylmethyl)-2-methyl-7-(tetrahydro-2H-pyran-4-yl)imidazo[5,1-f][1,2,4]triazin-4(3H)-on;
3-Methyl-2-(4-methylbenzyl)-7-(tetrahydro-2H-pyran-4-yl)imidazo[5,1-f][1,2,4]triazin-4(3H)-on;
3-Ethyl-2-(4-methylbenzyl)-7-(tetrahydro-2H-pyran-4-yl)imidazo[5,1-f][1,2,4]triazin-4(3H)-on;
2-(4-Methylbenzyl)-3-propyl-7-(tetrahydro-2H-pyran-4-yl)imidazo[5,1-f][1,2,4]triazin-4(3H)-on;
3-Isobutyl-2-(4-methylbenzyl)-7-(tetrahydro-2H-pyran-4-yl)imidazo[5,1-f][1,2,4]triazin-4(3H)-on;
3-(Cyclopentylmethyl)-2-(4-methylbenzyl)-7-(tetrahydro-2H-pyran-4-yl)imidazo[5,1-f][1,2,4]triazin-4(3H)-on;
3-(Cyclohexylmethyl)-2-(4-methylbenzyl)-7-(tetrahydro-2H-pyran-4-yl)imidazo[5,1-f][1,2,4]triazin-4(3H)-on;
3-(Cyclopropylmethyl)-2-(4-methylbenzyl)-7-(tetrahydro-2H-pyran-4-yl)imidazo[5,1-f][1,2,4]triazin-4(3H)-on;
2-(4-Methylbenzyl)-7-(tetrahydro-2H-pyran-4-yl)-3-((tetrahydro-2H-pyran-4-yl)methyl)imidazo[5,1-f][1,2,4]triazin-4(3H)-on;
3-(Cyclobutylmethyl)-2-(4-methylbenzyl)-7-(tetrahydro-2H-pyran-4-yl)imidazo[5,1-f][1,2,4]triazin-4(3H)-on;
cis-2-(4-Methylbenzyl)-3-((4-methylcyclohexyl)methyl)-7-(tetrahydro-2H-pyran-4-yl)imidazo[5,1-f][1,2,4]triazin-4(3H)-on;
trans-2-(4-Methylbenzyl)-3-((methylcyclohexyl)methyl)-7-(tetrahydro-2H-pyran-4-yl)imidazo[5,1-f][1,2,4]triazin-4(3H)-on;
(-)-2-(4-Methylbenzyl)-7-(tetrahydro-2H-pyran-4-yl)-3-((tetrahydrofuran-3-yl)methyl)imidazo[5,1-f][1,2,4]triazin-4(3H)-on;
(+)-2-(4-Methylbenzyl)-7-(tetrahydro-2H-pyran-4-yl)-3-((tetrahydrofuran-3-yl)methyl)imidazo[5,1-f][1,2,4]triazin-4(3H)-on;
3-(3-Fluorbenzyl)-2-methyl-7-(tetrahydro-2H-pyran-4-yl)imidazo[5,1-f][1,2,4]triazin-4(3H)-on;
3-(4-Fluorbenzyl)-2-methyl-7-(tetrahydro-2H-pyran-4-yl)imidazo[5,1-f][1,2,4]triazin-4(3H)-on;
3-Benzyl-2-methyl-7-(tetrahydro-2H-pyran-4-yl)imidazo[5,1-f][1,2,4]triazin-4(3H)-on;
3-(2-Chlorbenzyl)-2-methyl-7-(tetrahydro-2H-pyran-4-yl)imidazo[5,1-f][1,2,4]triazin-4(3H)-on;
3-(3-Chlorbenzyl)-2-methyl-7-(tetrahydro-2H-pyran-4-yl)imidazo[5,1-f][1,2,4]triazin-4(3H)-on;
2-Methyl-3-(3-methylbenzyl)-7-(tetrahydro-2H-pyran-4-yl)imidazo[5,1-f][1,2,4]triazin-4(3H)-on;
3-(3-Methoxybenzyl)-2-methyl-7-(tetrahydro-2H-pyran-4-yl)imidazo[5,1-f][1,2,4]triazin-4(3H)-on;
3-(2-Fluorbenzyl)-2-methyl-7-(tetrahydro-2H-pyran-4-yl)imidazo[5,1-f][1,2,4]triazin-4(3H)-on;
2-Methyl-3-(2-methylbenzyl)-7-(tetrahydro-2H-pyran-4-yl)imidazo[5,1-f][1,2,4]triazin-4(3H)-on;
2-Methyl-3-(4-methylbenzyl)-7-(tetrahydro-2H-pyran-4-yl)imidazo[5,1-f][1,2,4]triazin-4(3H)-on;
3-(4-Methoxybenzyl)-2-methyl-7-(tetrahydro-2H-pyran-4-yl)imidazo[5,1-f][1,2,4]triazin-4(3H)-on;
3-(4-Chlorbenzyl)-2-methyl-7-(tetrahydro-2H-pyran-4-yl)imidazo[5,1-f][1,2,4]triazin-4(3H)-on;
2-Ethyl-3-(3-fluorbenzyl)-7-(tetrahydro-2H-pyran-4-yl)imidazo[5,1-f][1,2,4]triazin-4(3H)-on;
3-(Cyclohexylmethyl)-7-(tetrahydro-2H-pyran-4-yl)imidazo[5,1-f][1,2,4]triazin-4(3H)-on;
3-(3-Fluorbenzyl)-7-(tetrahydro-2H-pyran-4-yl)imidazo[5,1-f][1,2,4]triazin-4(3H)-on;
3-(Cyclopentylmethyl)-7-(tetrahydro-2H-pyran-4-yl)imidazo[5,1-f][1,2,4]triazin-4(3H)-on;
3-(Cycloheptylmethyl)-7-(tetrahydro-2H-pyran-4-yl)imidazo[5,1-f][1,2,4]triazin-4(3H)-on;
und pharmazeutisch annehmbaren Salzen einer beliebigen dieser Verbindungen.

9. Verbindung nach einem der Ansprüche 1 bis 8 zur Verwendung als Medikament.

10. Pharmazeutische Zusammensetzung, umfassend eine therapeutisch wirksame Menge einer Verbindung nach einem der Ansprüche 1 bis 8 und einen oder mehrere pharmazeutisch annehmbare Träger, Verdünnungsmittel und Hilfsstoffe.

11. Verbindung nach einem der Ansprüche 1-8 zur Verwendung bei der Behandlung einer neurodegenerativen Störung, ausgewählt aus der Gruppe bestehend aus Morbus Alzheimer, Morbus Parkinson und Morbus Huntington, oder zur Behandlung einer psychiatrischen Störung, beispielsweise Aufmerksamkeitsdefizit-/Hyperaktivitätsstörung (ADHS), Depression, Angst, Narkolepsie, kognitive Beeinträchtigung und kognitive Beeinträchtigung in Verbindung mit Schizophrenie (CIAS), oder einer anderen Gehirnerkrankung wie Restless-Legs-Syndrom.

## Revendications

1. Composé selon la formule (I) : 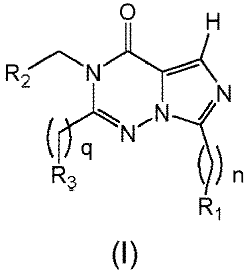 dans lequel :
n est 0 ou 1 ;
q est 0 ou 1 ;
R1 est choisi dans le groupe constitué d'un alkyle en C₁-C₈ linéaire ou ramifié, d'un cycloalkyle en C₃-C₈ monocyclique saturé, d'un tétrahydrofuranyle et d'un tétrahydropyranyle ; qui peuvent tous être substitués une ou plusieurs fois par du fluor ;
R2 est choisi dans le groupe constitué de l'hydrogène, d'un alkyle en C₁-C₈ linéaire ou ramifié, d'un cycloalkyle en C₃-C₈ monocyclique saturé, d'un tétrahydrofuranyle et d'un tétrahydropyranyle, ou
R2 est un phényle ou un hétéroaryle à 5 ou 6 chaînons, qui peuvent tous être substitués par un ou plusieurs substituants choisis dans le groupe constitué d'un halogène, d'un alkyle en C₁-C₃ et d'un méthoxy ; ou
R2 est un cycloalkyle en C₃-C₈ monocyclique saturé substitué une ou deux fois par un méthyle ;
R3 est choisi dans le groupe constitué de l'hydrogène, d'un alkyle en C₁-C₅ et d'un phényle ; ou
R3 est choisi dans le groupe constitué d'un alkyle en C₁-C₅ substitué une ou plusieurs fois par du fluor ; ou
R3 est un phényle substitué une ou plusieurs fois par un alkyle en C₁-C₃;
à condition que R2 et R3 ne puissent être de l'hydrogène dans la même molécule ;
et tautomères et sels d'addition pharmaceutiquement acceptables de ce composé.

2. Composé selon la revendication 1, dans lequel :
n est 0 et q est 0 ;
R1 est choisi parmi un tétrahydrofuranyle et un tétrahydropyranyle ;
R2 est choisi dans le groupe constitué d'un alkyle en C₁-C₈ linéaire ou ramifié, un phényle, un cycloalkyle en C₃-C₈ monocyclique saturé, un tétrahydrofuranyle et un tétrahydropyranile ; ou
R2 est choisi dans le groupe constitué d'un phényle substitué par un substituant choisi dans le groupe constitué d'un halogène, d'un alkyle en C₁-C₃ et d'un méthoxy ; et d'un cycloalkyle en C₃-C₈ monocyclique saturé substitué une ou deux fois par un méthyle ;
R3 est choisi dans le groupe constitué de l'hydrogène et d'un alkyle en C₁-C₅; ou
R3 est choisi parmi un méthyle substitué une, deux ou trois fois par du fluor; et d'un éthyle substitué une, deux ou trois fois par du fluor.

3. Composé selon la revendication 1, dans lequel n est 0 et R1 est un tétrahydropyranyle.

4. Composé selon la revendication 1, dans lequel R2 est un phényle.

5. Composé selon la revendication 1, dans lequel R2 est un phényle substitué par un substituant choisi dans le groupe constitué du fluor, du chlore et d'un méthyle.

6. Composé selon la revendication 1, dans lequel R3 est un méthyle ou un éthyle éventuellement substitué une, deux ou trois fois par du fluor.

7. Composé selon la revendication 1, dans lequel R3 est de l'hydrogène, un méthyle ou un éthyle ; et
R2 est choisi dans le groupe constitué d'un alkyle en C₁-C₈ linéaire ou ramifié, d'un cycloalkyle en C₃-C₈ monocyclique saturé, d'un tétrahydrofuranyle et d'un tétrahydropyranyle ; ou
R2 est un phényle ou un hétéroaryle à 5 ou 6 chaînons qui peuvent tous être substitués par un ou plusieurs substituants choisis dans le groupe constitué d'un halogène, d'un alkyle en C₁-C₃ et d'un méthoxy ; ou
R2 est un cycloalkyle en C₃-C₈ monocyclique saturé substitué une ou deux fois par un méthyle.

8. Composé selon la revendication 1, dans lequel le composé est choisi dans le groupe constitué des suivants :
3-(cyclohexylméthyl)-2-méthyl-7-(tétrahydro-2H-pyran-4-yl)imidazo[5,1-f][1,2,4]-triazin-4(3H)one ;
3-méthyl-2-(4-méthylbenzyl)-7-(tétrahydro-2H-pyran-4-yl)imidazo[5,1-f][1,2,4]-triazin-4(3H)one ;
3-éthyl-2-(4-méthylbenzyl)-7-(tétrahydro-2H-pyran-4-yl)imidazo[5,1-f][1,2,4]-triazin-4(3H)one ;
2-(4-méthylbenzyl)-3-propyl-7-(tétrahydro-2H-pyran-4-yl)imidazo[5,1-f][1,2,4]-triazin-4(3H)one ;
3-isobutyl-2-(4-méthylbenzyl)-7-(tétrahydro-2H-pyran-4-yl)imidazo[5,1-f][1,2,4]-triazin-4(3H)one ;
3-(cyclopentylméthyl)-2-(4-méthylbenzyl)-7-(tétrahydro-2H-pyran-4-yl)imidazo-[5,1-f][1,2,4]triazin-4(3H)one ;
3-(cyclohexylméthyl)-2-(4-méthylbenzyl)-7-(tétrahydro-2H-pyran-4-yl)imidazo-[5,1-f][1,2,4]triazin-4(3H)one ;
3-(cyclopropylméthyl)-2-(4-méthylbenzyl)-7-(tétrahydro-2H-pyran-4-yl)imidazo-[5,1-f][1,2,4]thazin-4(3H)one ;
2-(4-méthylbenzyl)-7-(tétrahydro-2H-pyran-4-yl)-3-((tétrahydro-2H-pyran-4-yl)-méthyl)imidazo[5,1-f][1,2,4]triazin-4(3H)one ;
3-(cyclobutylméthyl)-2-(4-méthylbenzyl)-7-(tétrahydro-2H-pyran-4-yl)imidazo-[5,1-f][1,2,4]triazin-4(3H)one ;
cis-2-(4-méthylbenzyl)-3-((4-méthylcyclohexyl)méthyl)-7-(tétrahydro-2H-pyran-4-yl)imidazo[5,1-f][1,2,4]triazin-4(3H)one ;
trans-2-(4-méthylbenzyl)-3-((4-méthylcyclohexyl)méthyl)-7-(tétrahydro-2H-pyran-4-yl)imidazo[5,1-f][1,2,4]triazin-4(3H)one ;
(-)-2-(4-méthylbenzyl)-7-(tétrahydro-2H-pyran-4-yl)-3-((tétrahydrofuran-3-yl)-méthyl)imidazo[5,1-f][1,2,4]triazin-4(3H)one ;
(+)-2-(4-méthylbenzyl)-7-(tétrahydro-2H-pyran-4-yl)-3-((tétrahydrofuran-3-yl)-méthyl)imidazo[5,1-f][1,2,4]triazin-4(3H)one ;
3-(3-fluorobenzyl)-2-méthyl-7-(tétrahydro-2H-pyran-4-yl)imidazo[5,1-f][1,2,4]-triazin-4(3H)one ;
3-(4-fluorobenzyl)-2-méthyl-7-(tétrahydro-2H-pyran-4-yl)imidazo[5,1-f][1,2,4]-triazin-4(3H)one ;
3-benzyl-2-méthyl-7-(tétrahydro-2H-pyran-4-yl)imidazo[5,1-f][1,2,4]triazin-4(3H)-one ;
3-(2-chlorobenzyl)-2-méthyl-7-(tétrahydro-2H-pyran-4-yl)imidazo[5,1-f][1,2,4]-triazin-4(3H)one ;
3-(3-chlorobenzyl)-2-méthyl-7-(tétrahydro-2H-pyran-4-yl)imidazo[5,1-f][1,2,4]-triazin-4(3H)one ;
2-méthyl-3-(3-méthylbenzyl)-7-(tétrahydro-2H-pyran-4-yl)imidazo[5,1-f][1,2,4]-triazin-4(3H)one ;
3-(3-méthoxybenzyl)-2-méthyl-7-(tétrahydro-2H-pyran-4-yl)imidazo[5,1-f][1,2,4]-triazin-4(3H)one ;
3-(2-fluorobenzyl)-2-méthyl-7-(tétrahydro-2H-pyran-4-yl)imidazo[5,1-f][1,2,4]-triazin-4(3H)one ;
2-méthyl-3-(2-méthylbenzyl)-7-(tétrahydro-2H-pyran-4-yl)imidazo[5,1-f][1,2,4]-triazin-4(3H)one ;
2-méthyl-3-(4-méthylbenzyl)-7-(tétrahydro-2H-pyran-4-yl)imidazo[5,1-f][1,2,4]-triazin-4(3H)one ;
3-(4-méthoxybenzyl)-2-méthyl-7-(tétrahydro-2H-pyran-4-yl)imidazo[5,1-f][1,2,4]-triazin-4(3H)one ;
3-(4-chlorobenzyl)-2-méthyl-7-(tétrahydro-2H-pyran-4-yl)imidazo[5,1-f][1,2,4]-triazin-4(3H)one ;
2-éthyl-3-(3-fluorobenzyl)-7-(tétrahydro-2H-pyran-4-yl)imidazo[5,1-f][1,2,4]-triazin-4(3H)one ;
3-(cyclohexylméthyl)-7-(tétrahydro-2H-pyran-4-yl)imidazo[5,1-f][1,2,4]triazin-4(3H)one ;
3-(3-fluorobenzyl)-7-(tétrahydro-2H-pyran-4-yl)imidazo[5,1-f][1,2,4]triazin-4(3H)-one ;
3-(cyclopentylméthyl)-7-(tétrahydro-2H-pyran-4-yl)imidazo[5,1-f][1,2,4]triazin-4(3H)one ; et
3-(cycloheptylméthyl)-7-(tétrahydro-2H-pyran-4-yl)imidazo[5,1-f][1,2,4]triazin-4(3H)one ;
et sels pharmaceutiquement acceptables de l'un quelconque de ces composés.

9. Composé selon l'une quelconque des revendications 1 à 8 pour utilisation comme médicament.

10. Composition pharmaceutique comprenant une quantité thérapeutiquement efficace d'un composé selon l'une quelconque des revendications 1 à 8 et un ou plusieurs véhicules, diluants et excipients pharmaceutiquement acceptables.

11. Composé selon l'une quelconque des revendications 1 à 8 pour utilisation dans le traitement d'un trouble neurodégénératif choisi dans le groupe constitué de la maladie d'Alzheimer, de la maladie de Parkinson et de la maladie d'Huntington ou pour le traitement d'un trouble psychiatrique tel qu'un trouble d'hyperactivité avec un déficit d'attention (ADHD), une dépression, une anxiété, une narcolepsie, un trouble cognitif et un trouble cognitif associé à la schizophrénie (CIAS) ou une autre maladie du cerveau tel que le syndrome des impatiences musculaires.
